# EUROPEAN PATENT SPECIFICATION

(11) **EP 2 443 252 B1**
(45) Date of publication and mention of the grant of the patent: **29.11.2017**
(21) Application number: 10725045.8
(22) Date of filing: 15.06.2010
(51) Int. Cl.: C12Q 1/68, G01N 33/574

(54) **BIOMARKERS AND METHODS FOR DETERMINING EFFICACY OF ANTI-EGFR ANTIBODIES IN CANCER THERAPY**
BIOMARKER UND VERFAHREN ZUR BESTIMMUNG DER WIRKSAMKEIT VON ANTI-EGFR-ANTIKÖRPERN IN DER KREBSTHERAPIE
BIOMARQUEURS ET PROCÉDÉS POUR DÉTERMINER L'EFFICACITÉ D'ANTICORPS ANTI-EGFR DANS UNE THÉRAPIE D'UN CANCER

(30) Priority: 19.06.2009 EP 09008042; 25.09.2009 EP 09012197
(43) Date of publication of application: 25.04.2012
(73) Proprietor: Merck Patent GmbH, 64293 Darmstadt (DE)
(72) Inventor: STROH, Christopher, 64807 Dieburg (DE); VON HEYDEBRECK, Anja, 64293 Darmstadt (DE)
(86) International application number: PCT/EP2010/003563
(87) International publication number: WO 2010/145796

(56) References cited:
- WO-A1-2009/140409
- WO-A2-2007/025044
- "GeneChip Human Genome Arrays", INTERNET CITATION, 12 June 2006 (2006-06-12), XP002384937, [retrieved on 2006-06-12]
- CROUSER ELLIOTT D ET AL: "Gene expression profiling identifies MMP-12 and ADAMDEC1 as potential pathogenic mediators of pulmonary sarcoidosis.", AMERICAN JOURNAL OF RESPIRATORY AND CRITICAL CARE MEDICINE 15 MAY 2009 LNKD- PUBMED:19218196, vol. 179, no. 10, 15 May 2009 (2009-05-15) , pages 929-938, XP002597071, ISSN: 1535-4970 & CROUSER ET AL: "Gene expression profiling identifies MMP-12 and ADAMDEC1 as potential pathogenic mediators of pulmonary sarcoidosis - on-line data supplement", AM. J. RESP. CRIT. CARE MED., vol. 179, 12 February 2009 (2009-02-12), pages 1-11, XP002597072,
- REYES PAZ A ET AL: "Apoptosis related genes expressed in cultured Fallopian tube epithelial cells infected in vitro with Neisseria gonorrhoeae.", BIOLOGICAL RESEARCH 2007 LNKD- PUBMED:18449459, vol. 40, no. 3, 2007, pages 319-327, XP002616085, ISSN: 0716-9760
- POLLARD S M ET AL: "Fibroblast growth factor induces a neural stem cell phenotype in foetal forebrain progenitors and during embryonic stem cell differentiation", MOLECULAR AND CELLULAR NEUROSCIENCES, SAN DIEGO, US, vol. 38, no. 3, 1 July 2008 (2008-07-01), pages 393-403, XP022777377, ISSN: 1044-7431, DOI: DOI:10.1016/J.MCN.2008.03.012 [retrieved on 2008-04-10]
- BAKER J.B. ET AL: "Evaluation of tumor gene expression and K-ras mutations in FFPE tumor tissue as predictors of response to cetuximab in metastatic colorectal cancer", JOURNAL OF CLINICAL ONCOLOGY, vol. 26, no. 15S, 3512, 20 May 2008 (2008-05-20), XP002597049,
- TEJPAR S ET AL: "High amphiregulin and epiregulin expression in KRAS wild type colerectal primaries predicts response and survival benefit after treatment with cetuximab and irinotecan for metastatic disease", 2008 GASTROINTESTINAL CANCERS SYMPOSIUM, ASCO, US, 1 January 2008 (2008-01-01), pages 1-3, XP003026155,
- KHAMBATA-FORD S ET AL: "Expression of epiregulin and amphiregulin and K-ras mutation status predict disease control in metastatic colorectal cancer patients treated with cetuximab", JOURNAL OF CLINICAL ONCOLOGY, vol. 25, no. 22, 1 August 2007 (2007-08-01), pages 3230-3237, XP002493882, ISSN: 0732-183X
- HARBISON C T ET AL: "In reply", JOURNAL OF CLINICAL ONCOLOGY 2008 US LNKD- DOI:10.1200/JCO.2008.16.0473, vol. 26, no. 13, 2008, pages 2230-2231, XP002597050, ISSN: 0732-183X
- LOUPAKIS FOTIOS ET AL: "PTEN expression and KRAS mutations on primary tumors and metastases in the prediction of benefit from cetuximab plus irinotecan for patients with metastatic colorectal cancer", JOURNAL OF CLINICAL ONCOLOGY, vol. 27, no. 16, 27 April 2009 (2009-04-27), pages 2622-2629, XP009121162, ISSN: 0732-183X
- FRATTINI M ET AL: "PTEN loss of expression predicts cetuximab efficacy in metastatic colorectal cancer patients", CANCER RESEARCH, vol. 97, no. 8, 22 October 2007 (2007-10-22), pages 1139-1145, XP002547206, ISSN: 1538-7445 [retrieved on 2007-10-16]
- ALLEGRA CARMEN J ET AL: "American Society of Clinical Oncology provisional clinical opinion: testing for KRAS gene mutations in patients with metastatic colorectal carcinoma to predict response to anti-epidermal growth factor receptor monoclonal antibody therapy.", JOURNAL OF CLINICAL ONCOLOGY, vol. 27, no. 12, 20 April 2009 (2009-04-20), pages 2091-2096, XP002597052, ISSN: 1527-7755
- KHAMBATA-FORD S. ET AL: "K-Ras mutation (mut), EGFR-related, and exploratory markers as response predictors of cetuximab in first-line advanced NSCLC: Retrospective analyses of the BMS099 trial", JOURNAL OF CLINICAL ONCOLOGY, ASCO ANNUAL MEETING PROCEEDINGS (POST-MEETING EDITION), 20 May 2009 (2009-05-20), XP002597053,
- TABERNERO JOSEP ET AL: "Pharmacogenomic and pharmacoproteomic studies of cetuximab in metastatic colorectal cancer: biomarker analysis of a phase I dose-escalation study.", JOURNAL OF CLINICAL ONCOLOGY, vol. 28, no. 7, 1 March 2010 (2010-03-01), pages 1181-1189, XP008125565, ISSN: 1527-7755

## Description

### FIELD OF THE INVENTION:

The invention relates to biomarkers based on genes or gene expression products and methods for determining the efficacy of anti-EGFR antibodies in the treatment of EGFR expressing cancer. The invention is further related to the prediction of sensitivity or resistance of a patient suffering from EGFR expressing cancer to the treatment of said patient with a specific anti-EGFR antibody. The invention is preferably related to the identification of respective biomarkers that allow a better prediction of the clinical outcome of the treatment with anti-EGFR antibodies in patients with KRAS wild-type tumors. In this context, the invention especially relates to anti-EFGR antibody c225/cetuximab (Erbitux®) and its use in patients suffering especially from colorectal cancer (CRC).

### BACKGROUND OF THE INVENTION

Monoclonal antibodies are commonly used in the treatment of cancer. Since antibodies are expensive drugs charging the national health care authorities, and often cause undesired side-effects, which provoke an additional burden and stress for the patient suffering from a severe and often terminal disease, it would be very desirable to know in advance whether treating a specific patient with a specific antibody drug could really improve or even cure the patient's condition. There are already a couple of clinical and histological parameters which are used to obtain a prognosis whether a specific drug and / or treatment regimen may be successful for treating a disease. However, it has been shown that tumors very often elicit a diverse genetic pattern, which may change from one individual to another one. Thus, a specific drug or a specific treatment, which may improve the clinical condition of a first patient, is less or not effective in a second patient suffering from the same cancer. For example, patients suffering from colorectal cancer may respond to a certain specific antibody drug differently. In the worst case a specific patient group does not respond at all to the drug, whereas another group of patients elicit a satisfying therapeutic response thereto dependent on a different genetic tumor pattern and disposition.

Although modern molecular biology and biochemistry have revealed hundreds of genes whose activities influence the behavior of tumor cells, the state of their differentiation, and their sensitivity or resistance to certain therapeutic drugs, such as antibodies, the status of these genes usually has not been exploited for the purpose of routinely making clinical decisions about drug treatments. Exception are the use of ErbB2 (Her2) protein expression in breast carcinomas to select patients with the Her2 antagonist drug Herceptin® (Genentech) Another exception is the finding that mutations in the KRAS gene of EGFR expressing tumors are associated with the lack of sensitivity or response to anti-EGFR antibodies (Allegra et al. 2009, J Clin Oncol [Epub ahead of print]).

New prognostic and predictive markers, which would facilitate a selection of patients for therapy , are needed to better predict patient response to treatments, such as small molecule or biological molecule drugs, in the clinic. The classification of patient samples is a crucial aspect of cancer diagnosis and treatment. The association of a patient's response to a treatment with molecular and genetic markers can open up new opportunities for treatment development in non-responding patients, or distinguish a treatment's indication among other treatment choices because of higher confidence in the efficacy. Further, the pre-selection of patients who are likely to respond well to a drug, or a combination of drugs or a specific regimen may reduce the number of patients needed in a clinical study or accelerate the time needed to complete a clinical development program.

The epidermal growth factor receptor (EGFR) and its downstream signaling effectors, notably members of the Ras/Raf/MAP kinase pathway, play an important role in both normal and malignant epithelial cell biology (Normanno et al., Gene 366, 2-16 (2006)) and have therefore become established targets for therapeutic development.

Several monoclonal chimeric, humanized or fully human monoclonal antibodies have been developed which recognize and inhibit the EGFR. Two examples of antibodies, which have been marketed in meantime are cetuximab (ERBITUX®) and panitumumab (VECTIBIX®)

The anti-EGFR antibody c225 (cetuximab), a chimeric IgG1, which was demonstrated to inhibit EGF-mediated tumor cell growth in vitro and to inhibit human colorectal tumor growth in vivo, received marked approval in 2003. Its sequence was first disclosed in WO 96/40210. The antibody as well as in general all anti-EGFR antibodies, appear to act, above all, in synergy with certain chemotherapeutic agents (i.e., doxorubicin, adriamycin, taxol, and cisplatin) to eradicate human tumors in vivo in xenograft mouse models (e.g. EP 0667165). Furthermore, it could be shown that the combination of the anti-EGFR antibody c225 with a second humanized anti-EGFR antibody matuzumab (Mab h425) shows a synergistic effect in vitro models, indicating that these two antibodies although directed to the same receptor bind to different epitopes on the receptor (WO 2004/032960).

It was shown in the past that about 75% of patients treated with anti-EGFR antibodies, including cetuximab, or respective small molecule inhibitors develop more or less severe skin rash very fast after starting treatment. Although frequently tolerable and manageable, approximately 10% of patients require dose interruption and/or dose reduction due to the severe symptoms, and a few patients discontinue therapy. The increasingly apparent association of cutaneous toxicity with favorable clinical outcomes to EGFR inhibitors makes "achievement" of rash a desirable but potentially troublesome toxicity in patients experiencing therapeutic benefit, sometimes limiting the utility of these agents. Nonetheless, the occurrence of skin rash during anti-EGFR antibody treatment can be taken as reliable surrogate marker for therapeutic response to the treatment with said antibody, for example, cetuximab.

However, selecting skin rash occurrence as surrogate marker is not optimal because identification of cancer patients that generally do not respond to the treatment with an anti-EGFR antibodies is possible not before having administered the drug to the patient for a longer time.

Therefore, the finding that mutation of the KRAS gene (codon 12/13) and gene product in EGFR expressing tumor is responsible for insensitivity to the treatment of metastatic colorectal cancer with EGFR inhibitors can be regarded as improvement in the prediction whether a treatment is successful or not. WO 2008/112269 reports that panitumumab, a human Anti-EGFR antibody is effective only in KRAS wild-type tumor patients. Khambata-Ford et al. (2007, J. Clin. Oncol. 25, 3230) describe that metastatic colorectal cancer patients with tumors that have high gene expression levels of epiregulin and amphiregulin, as well as patients with wild-type KRAS tumors are more likely to have disease control on cetuximab treatment.

Baker et al. (J. of Clinical Oncology, Vol. 26, No. 15S, 3512, 20 May 2008) discloses the evaluation of tumor gene expression and K-RAS mutations in FFPE tumor tissue as predictors of response to cetuximab in metastatic colorectal cancer.

Tejpar et al (Gastrointestinal Cancer Symposium, ASCO, US, 1 January 2008, p- 1-3) discloses high amphiregulin and epiregulin expression in KRAS wild type colorectal primaries predicts response and survival benefit after treatment with cetuximab and irinotecan for metastatic disease.

Harbinson et al ("in reply" J. of Clinical Oncology 2008 US Vol. 26, No. 13, pages 2230-2231) in the publication "Expression of Epiregulin and Amphiregulin and KRAS mutation status predict disease control in metastatic colorectal cancer patients treated with cetuximab" addresses the predictive value of KRAS.

WO 2007/025044 A2 discloses biomarkers and methods for determining sensitivity to epidermal growth factor receptor modulators.

Despite above-said recent advances, a major challenge in cancer treatment remains to select patients for specific treatment regimens based on pathogenetic and / or genetic markers in order to optimize outcome. In context of treating EGFR expressing tumors with anti-EGFR antibodies inhibiting growth of these tumors, it would be helpful to know and better understand which patients are able to respond to an intended treatment with these antibodies, especially in view of newer findings that even in the KRAS-wild type tumor group not all patients (ca. 40%) do respond well to the treatment of anti-EGFR antibodies and other EGFR inhibitors.

Therefore, a need exists for diagnostic tests, methods and tools using biomarkers that simultaneously can provide predictive information about patient responses to the variety of treatment options including personally different tumor genotypes.

### SUMMARY OF THE INVENTION

The invention discloses predictive biomarkers for determining the efficacy of an anti-EGFR antibody in the treatment of cancer.

In one embodiment of the invention specific biomarkers are described which can be used to predict before administration in a patient the efficacy of an anti-EGFR antibody in the treatment of tumors in KRAS wild-type patients or patients having a mutation in the KRAS gene.

In a further embodiment of the invention specific biomarkers are disclosed which can be used to predict more exactly the efficacy or the degree of efficacy of an anti-EGFR antibody in the treatment of tumors in patients having no mutation in the KRAS gene (KRAS wild type), which usually respond statistically but not necessarily individually positive to an anti-EGFR antibody therapy.

Another embodiment of the invention is related to biomarkers which indicate a high likelihood of a good (positive biomarkers) or a bad (negative biomarkers) response to an anti-EGFR antibody treatment in patients suffering from colorectal cancer (CRC), preferably metastatic colorectal cancer (mCRC), squamous cell head and neck cancer (SCCHN) or non-small cell lung cancer (NSCLC).

In a specific embodiment of the invention biomarkers are disclosed which are predictive for the efficacy or non-efficacy of a tumor related (solid or metastatic tumors) therapy with the anti-EGFR antibody cetuximab (Erbitux®).

In a preferred embodiment of the invention are disclosed which are predictive for the efficacy or non-efficacy of a tumor related (solid or metastatic tumors) therapy with the anti-EGFR antibody cetuximab (Erbitux®), wherein the tumor is CRC, mCRC, SCCHN or NSCLC.

In a preferred embodiment of the invention are disclosed which are predictive for the efficacy or non-efficacy of a tumor related (solid or metastatic tumors) therapy with the anti-EGFR antibody cetuximab (Erbitux®), wherein the tumor is CRC, mCRC, SCCHN or NSCLC, and wherein the patients suffering from said cancers preferably show an individual KRAS wild-type gene pattern.

In another embodiment the invention relates to an *in vitro* method for predicting by diagnostic means and / or diagnostic apparatus the likelihood that a patient suffering from KRAS wild type EGFR expressing tumor, who is a candidate for treatment with an EGFR antibody, will respond or not respond to the treatment with said anti-EGFR antibody.

According to the invention the method comprises determining the expression level of one or more prognostic genes or gene expression products thereof in a tissue sample obtained from said patient, wherein high or low expression of the gene/gene product compared to a clinical relevant reference value indicates that the patient is likely to respond to said treatment or is likely not to respond to the treatment.

Genes or gene products causing high expression in a sample of a tumor patient that has a high likelihood to respond or do respond to the treatment with an anti-EGFR antibody, preferably cetuximab are according to the invention selected from the group of genes consisting of: ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL,NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, and SHROOM2.

Genes or gene products causing high expression in a sample of a tumor patient that has a low likelihood to respond or do not respond to the treatment with an anti-EGFR antibody, preferably cetuximab, are according to the invention selected from the group of genes consisting of: C7orf46, CAST, DCP2, DIP2B, ERAP1, INSIG2, KIF21A, KLK6, NGRN, NRIP1, PHGDH, PPP1R9A, QPCT, RABEP1, RPA1,RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, and UBE2K, indicates that the patient is likely not to respond to said treatment compared to a reference value.

In another embodiment of the invention preferred biomarkers which are above-average expressed and indicate that the patient will likely respond or will likely respond superior to the treatment with the respective anti-EGFR antibody (e.g. cetuximab), are selected from the group consisting of EPDR1, KCNK5, KHDRBS3, PGM2L1, SHROOM2, STK38, RGMB, SPIRE2 and VAV3 or the respective gene expression products of said group.

In another embodiment of the invention preferred biomarkers which are above-average expressed and indicate that the patient will not likely respond or will not likely respond superior to the treatment with the respective anti-EGFR antibody (e.g. cetuximab), are selected from the group consisting of ASB6, ATM, BMI1, CDC42EP2, EDEM3, PLLP, RALBP1, SLC4A11, TNFSF15, TPK1, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, and UBE2K or the respective gene expression products of said group.

In a further embodiment the preferred biomarker according to the invention predictive for a positive response of the patient to an anti-EGFR antibody is VAV3 and the preferred biomarker according to the invention predictive for a negative or negligible response of the patient to an anti-EGFR antibody is TGFa.

In another embodiment a respective method is applied, wherein at least one considerably or highly expressed first biomarker as specified above and in the claims is used which indicates that the patient will probably respond well or extraordinary or superior to the treatment with the anti-EGFR antibody preferably cetuximab (compared to a clinical average or standard response and / or expression value calculated from a respective average patient cohort), and at least one considerably or highly expressed second biomarker as specified above and in the claims is used which indicates that the patient probably will not respond well or extraordinary or superior to the treatment with the anti-EGFR antibody preferably cetuximab (compared to a clinical average or standard response and / or expression value calculated from a respective average patient cohort).

In another embodiment a respective method is applied for an *in vitro* method for predicting the likelihood that a patient suffering from KRAS wild type EGFR expressing tumor and is a candidate for treatment with an EGFR antibody, will respond to the treatment with said anti-EGFR antibody, wherein expression levels of one or more of the above and below specified biomarkers are determined in combination with a AREG and / or EREG in context with the treatment of a tumor patient with an anti-EGFR antibody, preferably cetuximab.

Preferably, a respective method is applied, wherein the gene or gene product expression levels of VAV3 and ARAG or EREG are determined in context with the treatment of a tumor patient, preferably a CRC or mCRC tumor patient, with an anti-EGFR antibody, preferably cetuximab.

In a further specific embodiment, a respective method is applied, wherein gene or gene product expression levels of VAV3 and ARAG or EREG are determined in context with the treatment of a tumor patient, preferably a CRC or mCRC tumor patient, with an anti-EGFR antibody, preferably cetuximab.

In another preferred embodiment according to the invention, a respective method is applied, wherein gene or gene product expression levels TGFa and ARAG or EREG are determined in context with the treatment of a tumor patient, preferably a CRC or mCRC tumor patient, with an anti-EGFR antibody, preferably cetuximab.

In another preferred embodiment according to the invention, a respective method is applied, wherein gene or gene product expression levels VAV3 and TGFa determined in context with the treatment of a tumor patient, preferably a CRC or mCRC tumor patient, with an anti-EGFR antibody, preferably cetuximab.

In another preferred embodiment according to the invention, a respective method is applied, wherein gene or gene product expression levels VAV3, TGFa and ARAG or EREG are determined in context with the treatment of a tumor patient, preferably a CRC or mCRC tumor patient, with an anti-EGFR antibody, preferably cetuximab.

In a further aspect, the invention relates to an *in vitro* method for predicting the likelihood that a patient suffering from KRAS wild type EGFR expressing cancer will respond therapeutically to the treatment with an anti-EGFR antibody, preferably cetuximab, the method comprises:
(a) measuring by diagnostic means and / or diagnostic apparatus in a biopsy tissue sample from tumor tissue or plasma of said patient the expression level of biomarkers selected from the group (i) consisting of ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, SHROOM2, and / or from group (ii) consisting of C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, and UBE2K,
(b) exposing ex-vivo a tissue sample from tumor or plasma of said patient to said anti-EGFR antibody, (c) measuring again in said exposed tissue sample of step (b) the expression level of one or more biomarkers specified in step (a), and (d) calculating the differences in expression levels measured in steps(b) and (c),
wherein an increase in the expression level of the biomarkers of group (i) obtained in step (c) compared to step (a) indicates an increased likelihood that said patient responds therapeutically to the treatment with said anti-EGFR antibody, and wherein an increase in the expression level of the biomarkers of group (ii) obtained in step (c) compared to step (a) indicates a decreased likelihood that said patient responds therapeutically to the treatment with said anti-EGFR antibody.

In another aspect, the invention relates to a respective *in vitro* method as disclosed above and below, wherein the patient does not only suffer from KRAS wild type EGFR expressing tumor but in addition shows a mutation in the EGFR gene of the tumor tissue. In a specific embodiment this mutation is responsible for skin rash associated with the administration of the anti-EGFR antibody, preferably cetuximab. This mutation causes preferably a R521K polymorphism in EGFR.

### SHORT DESCRIPTION OF THE FIGURES:

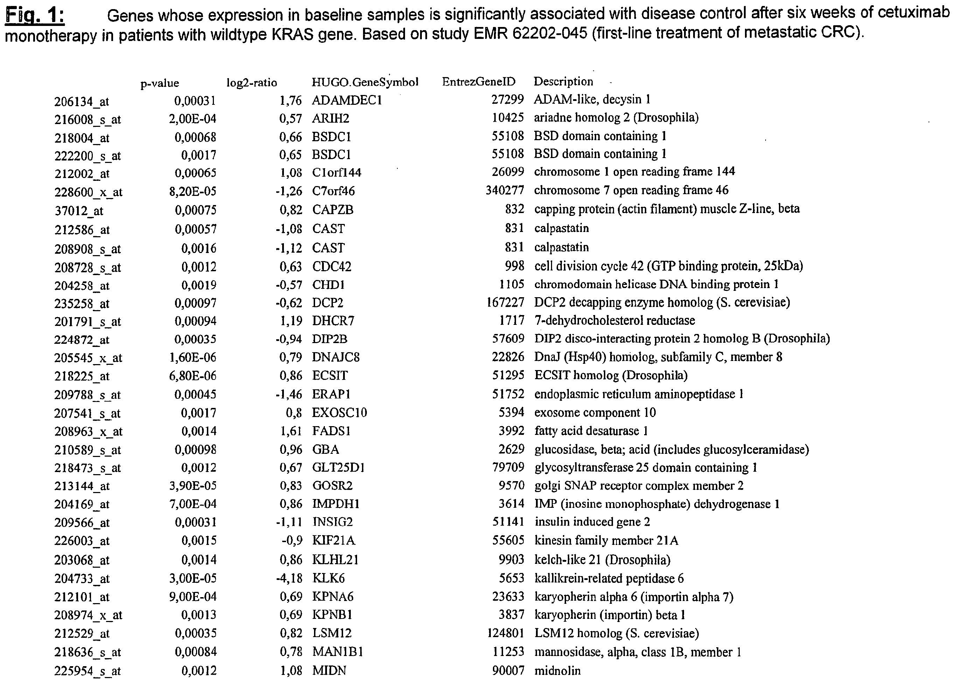

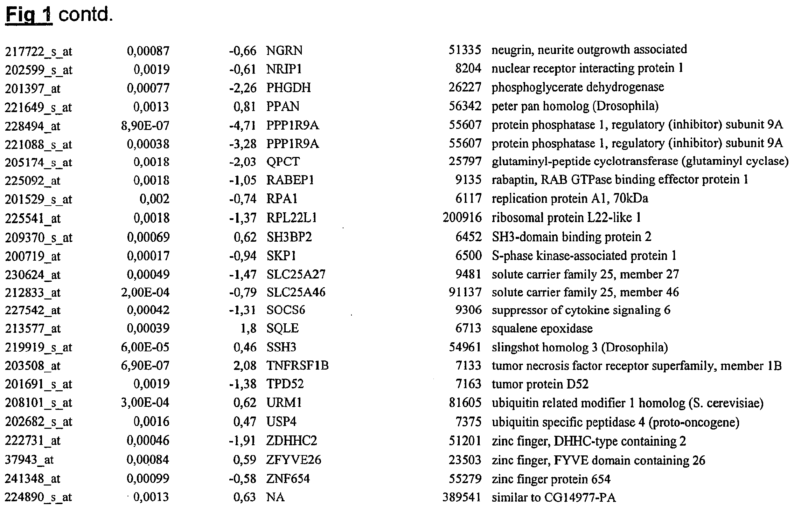

**Fig. 1****:** Genes whose expression in baseline samples is significantly associated with disease control after six weeks of cetuximab monotherapy in patients with wildtype KRAS gene (p < 0.002, moderated t-test). Based on study EMR 62202-045 (first-line treatment of metastatic CRC)

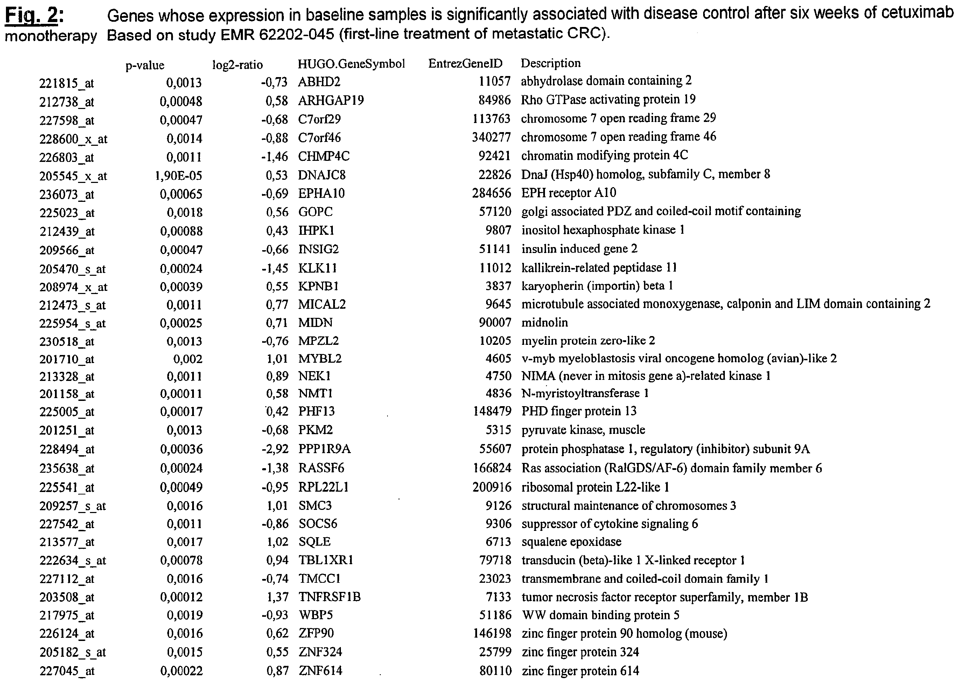

**Fig. 2****:** Genes whose expression in baseline samples is significantly associated with disease control after six weeks of cetuximab monotherapy (p < 0.002, moderated t-test). Based on study EMR 62202-045 (first-line treatment of metastatic CRC).

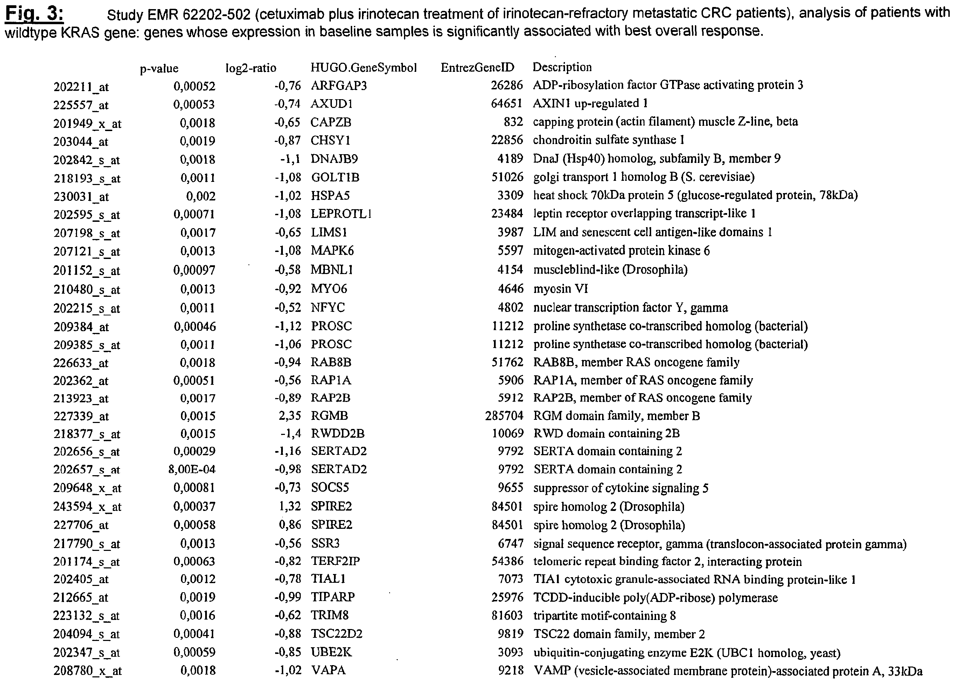

**Fig. 3****:** Study EMR 62202-502 (cetuximab plus irinotecan treatment of irinotecan-refractory metastatic CRC patients), analysis of patients with wildtype KRAS gene: genes whose expression in baseline samples is significantly associated with best overall response (p < 0.002, Welch t-test)

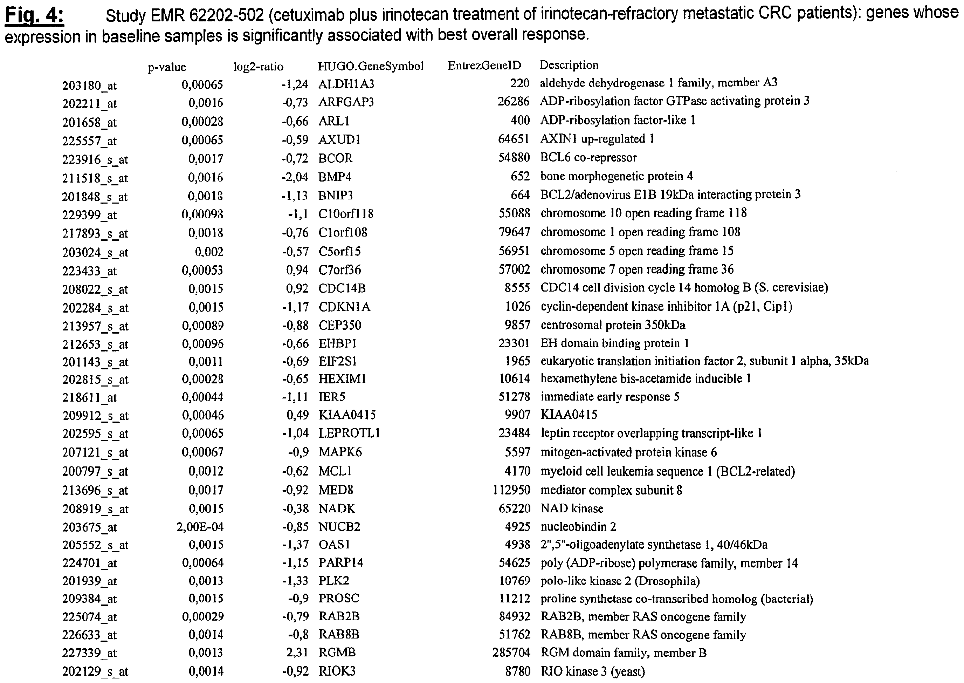

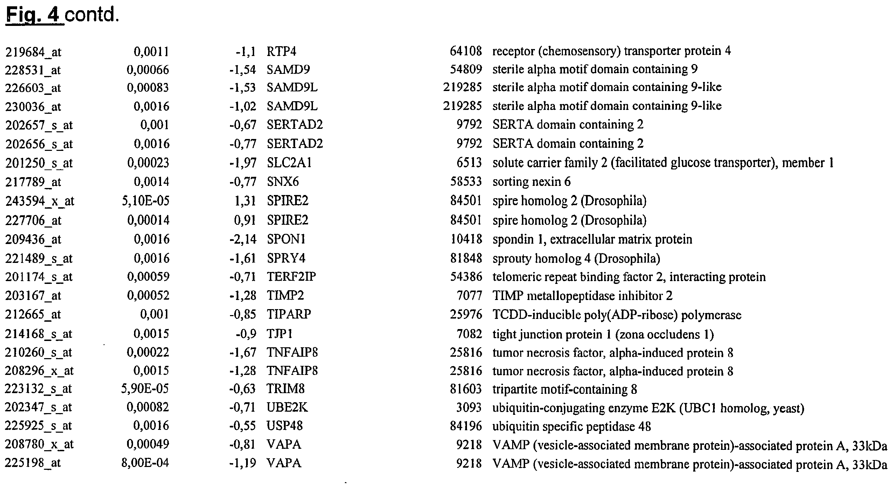

**Fig. 4****:** Study EMR 62202-502 (cetuximab plus irinotecan treatment of irinotecan-refractory metastatic CRC patients): genes whose expression in baseline samples is significantly associated with best overall response (p < 0.002, Welch t-test).

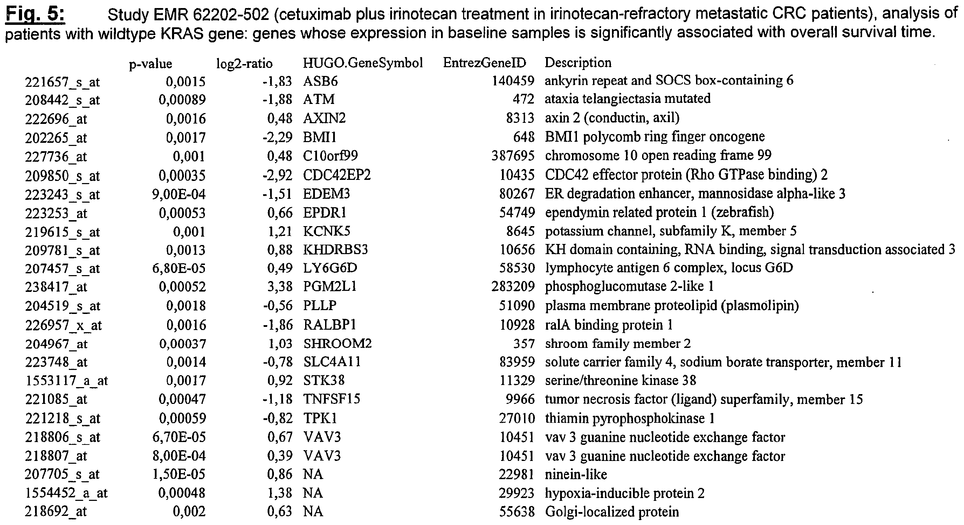

**Fig. 5****:** Study EMR 62202-502 (cetuximab plus irinotecan treatment in irinotecan-refractory metastatic CRC patients), analysis of patients with wildtype KRAS gene: genes whose expression in baseline samples is significantly associated with overall survival time (p < 0.002, Cox proportional hazards regression)

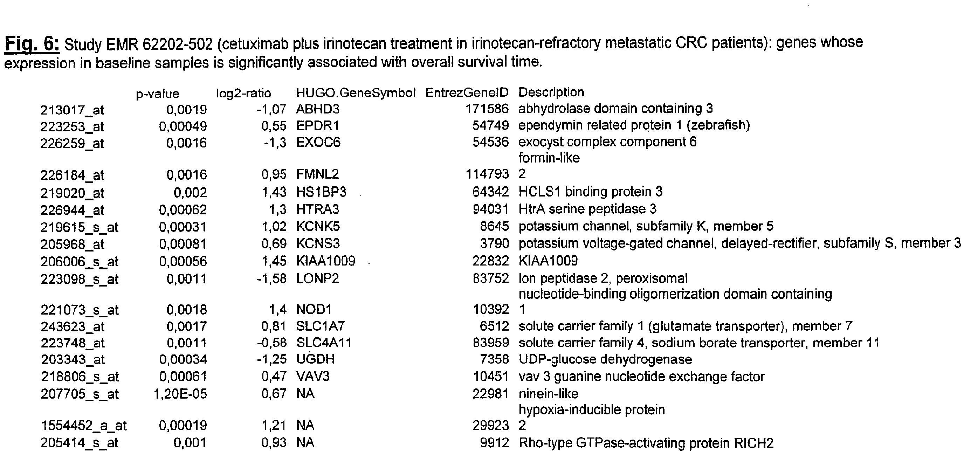

**Fig. 6****:** Study EMR 62202-502 (cetuximab plus irinotecan treatment in irinotecan-refractory metastatic CRC patients): genes whose expression in baseline samples is significantly associated with overall survival time (p < 0.002, Cox proportional hazards regression)

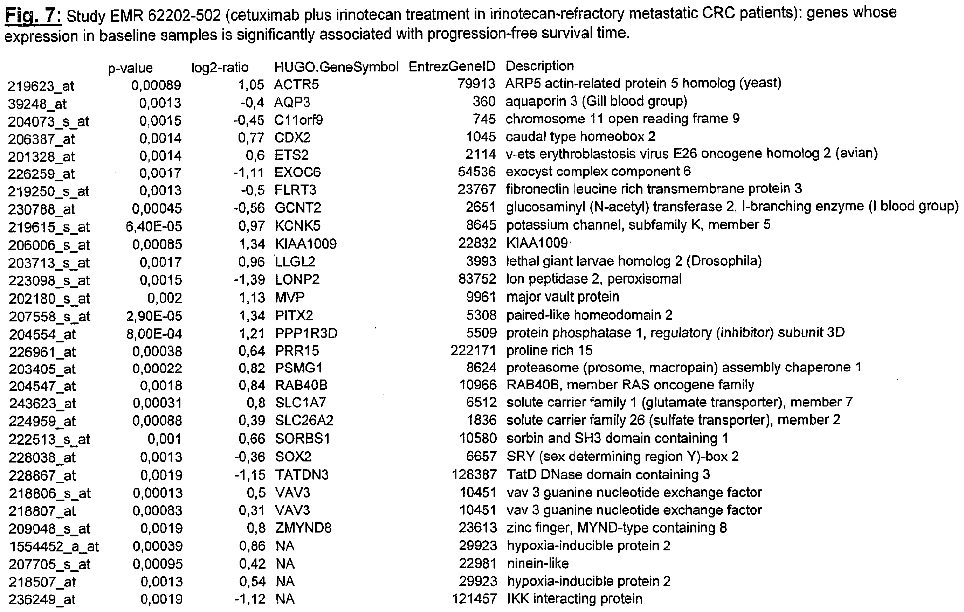

**Fig. 7****:** Study EMR 62202-502 (cetuximab plus irinotecan treatment in irinotecan-refractory metastatic CRC patients): genes whose expression in baseline samples is significantly associated with progression-free survival time (p < 0.002, Cox proportional hazards regression).

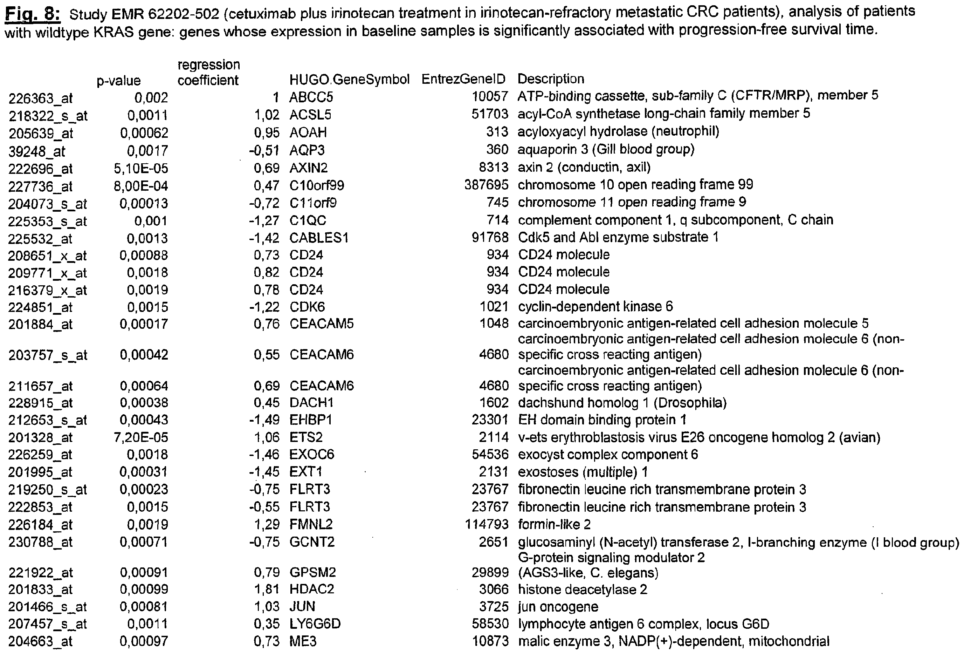

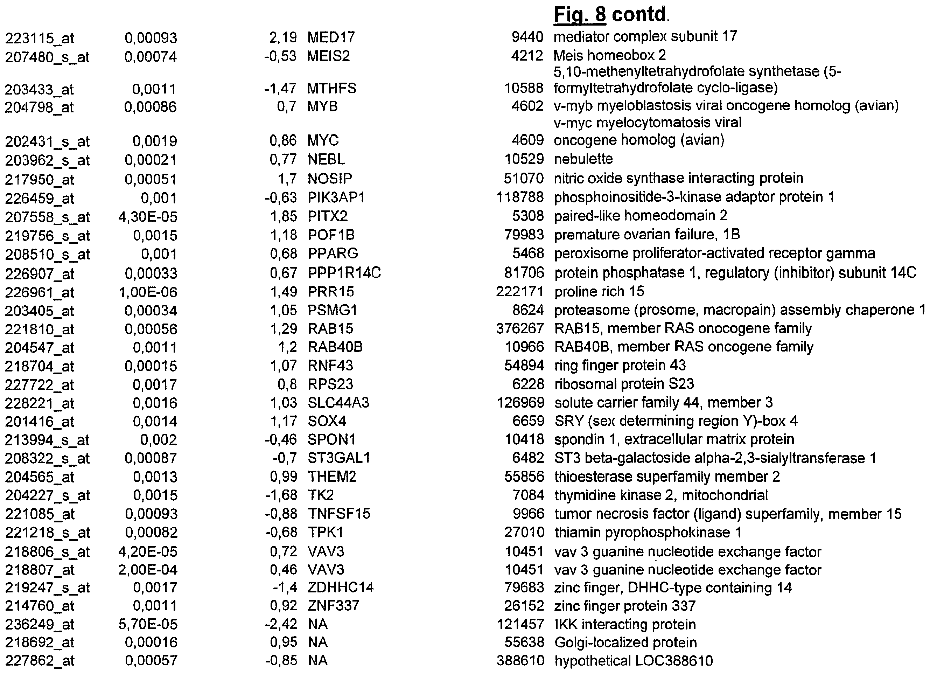

**Fig. 8****:** Study EMR 62202-502 (cetuximab plus irinotecan treatment in irinotecan-refractory metastatic CRC patients), analysis of patients with wildtype KRAS gene: genes whose expression in baseline samples is significantly associated with progression-free survival time (p < 0.002, Cox proportional hazards regression)

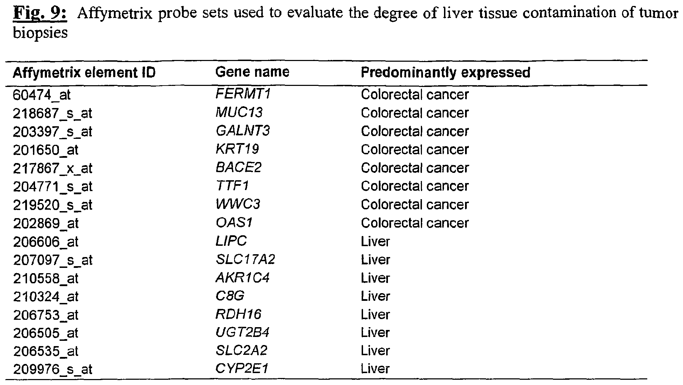

**Fig. 9****:** Affymetrix probe sets used to evaluate the degree of liver tissue contamination of tumor biopsies.

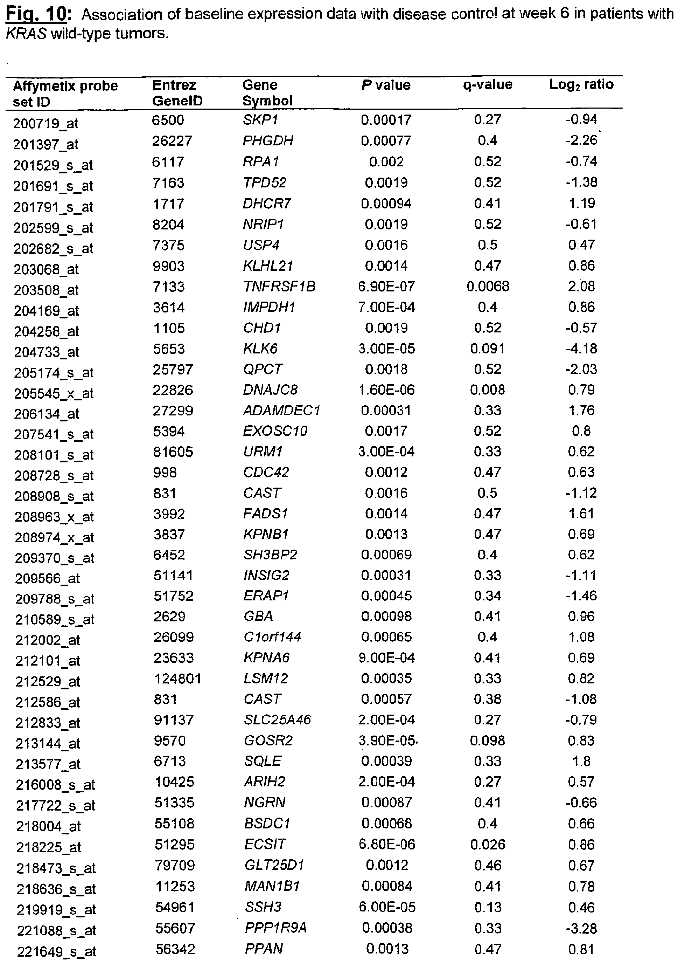

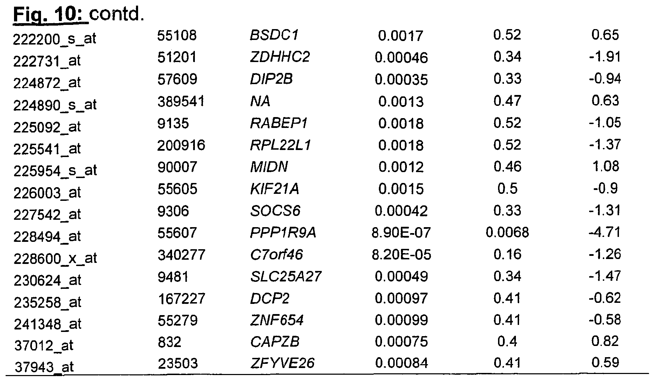

**Fig. 10****:** Association of baseline expression data with disease control at week 6 in patients with *KRAS* wild-type tumors: 57 probe sets with *P* <.002. Log-ratio values are mean log₂ expression levels of patients with disease control minus those of patients with progressive disease, adjusted for the degree of liver contamination of the samples.

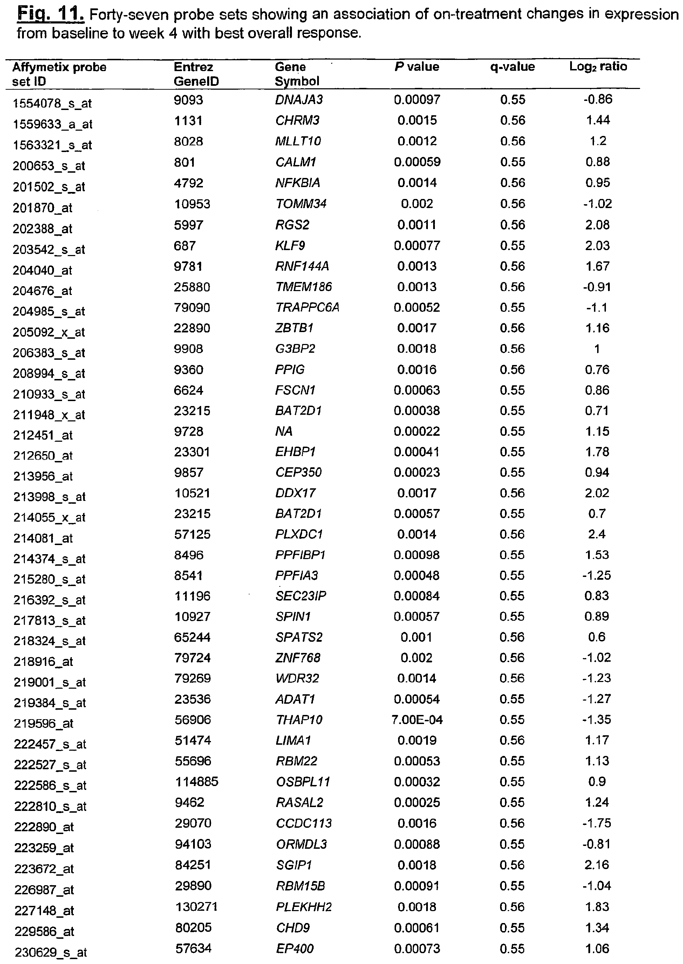

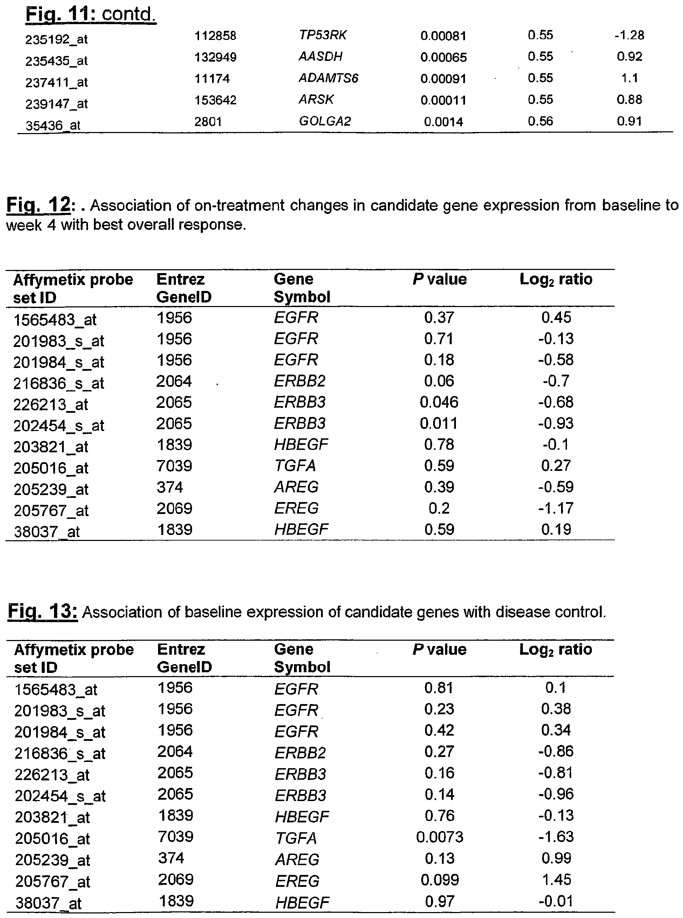

**Fig. 11****:** Forty-seven probe sets showing an association of on-treatment changes in expression from baseline to week 4 with best overall response. Log-ratio values represent means of on-treatment changes of patients with partial response minus those of patients with stable or progressive disease, adjusted for the degree of liver contamination of the samples.
**Fig. 12****:** Association of on-treatment changes in candidate gene expression from baseline to week 4 with best overall response. Log-ratio values represent means of on-treatment changes of patients with partial response minus those of patients with stable or progressive disease, adjusted for the degree of liver contamination of the samples.
**Fig. 13****:** Association of baseline expression of candidate genes with disease control at week 6 in tumors with *KRAS* wild-type status. Log-ratio values are mean log₂ expression levels of patients with disease control minus those of patients with progressive disease, adjusted for the degree of liver contamination of the samples.

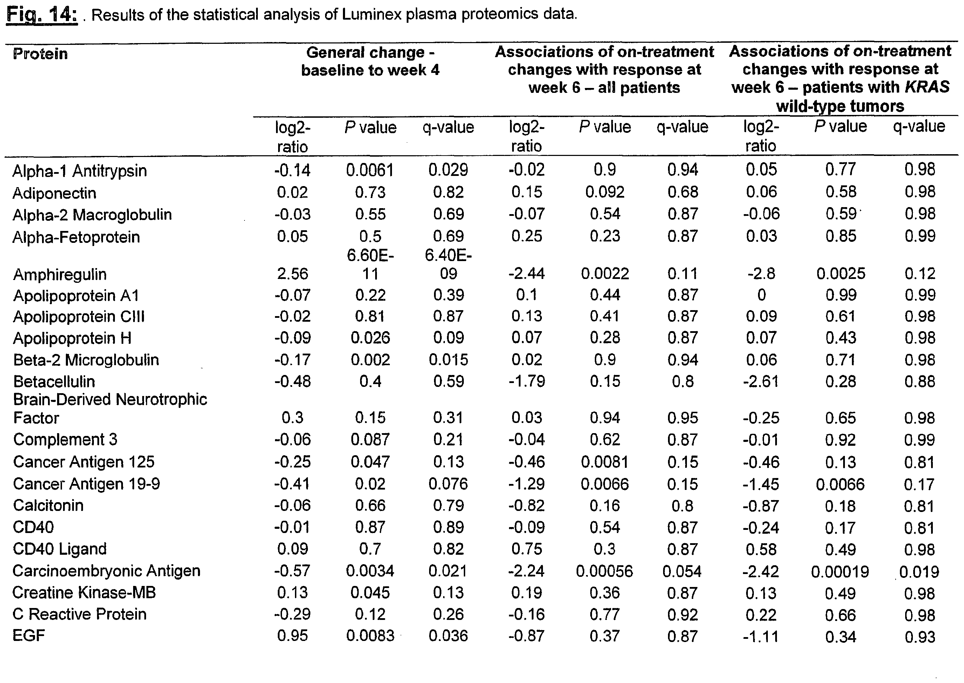

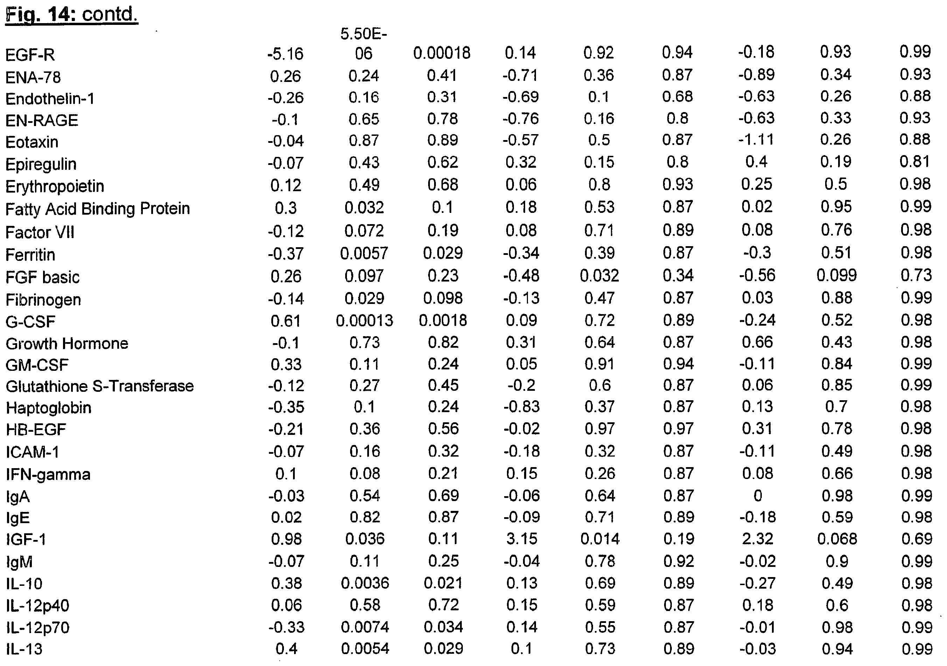

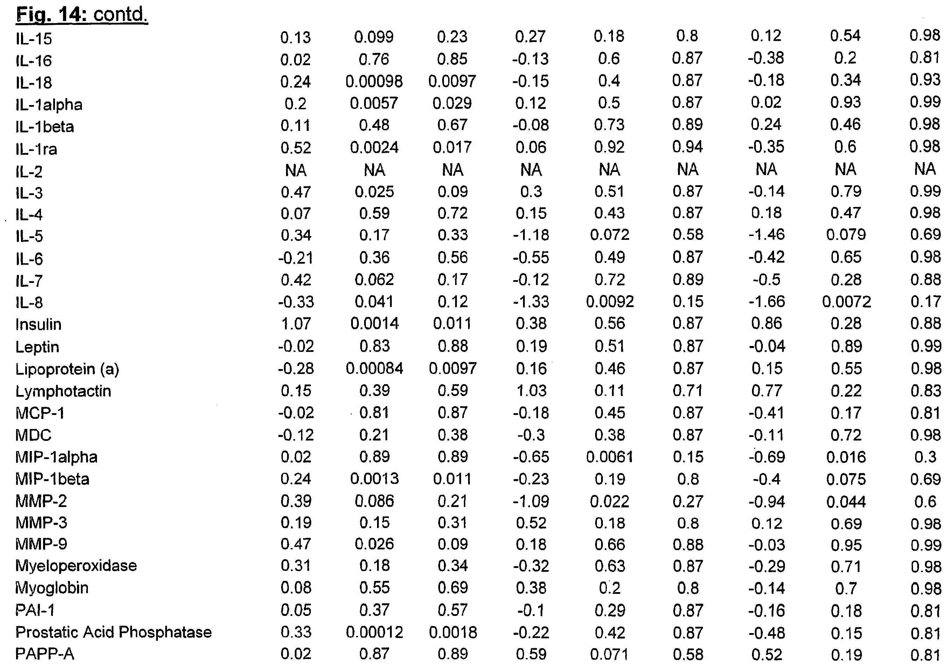

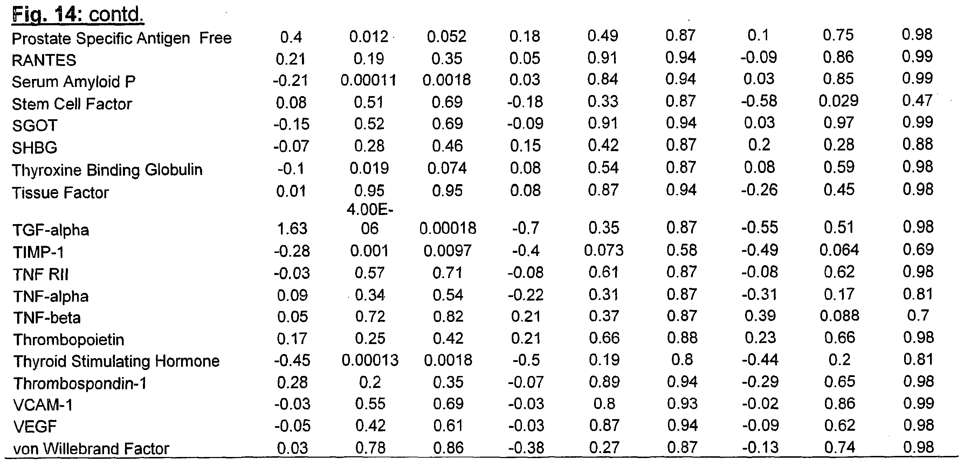

**Fig. 14****:** . Results of the statistical analysis of Luminex plasma proteomics data. Analyses refer to general changes between baseline and week 4 samples, associations of on-treatment changes with response at week 6 among all patients, as well as among the patients with *KRAS* wild-type tumors. For each of these analyses, log₂-ratio values, p-values and q-values are given for each measured protein. Log2-ratio values refer to the mean difference of log₂ concentrations between week 4 and baseline samples (general change), or to the difference between these mean differences between responders and non-responders (association with response).

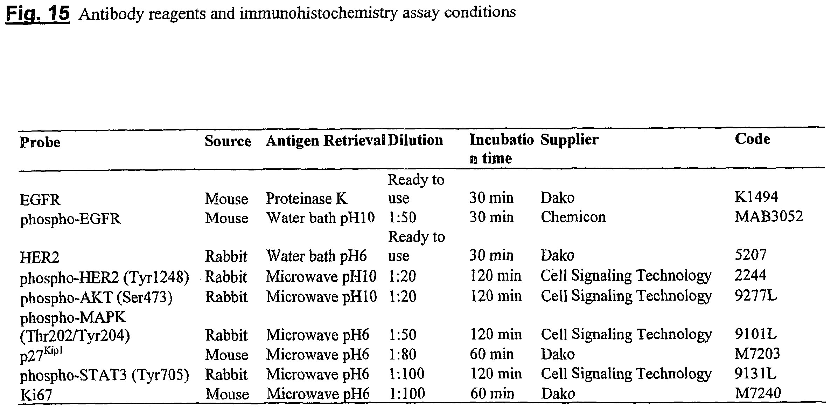

**Fig. 15****:** Antibody reagents and immunohistochemistry assay conditions.

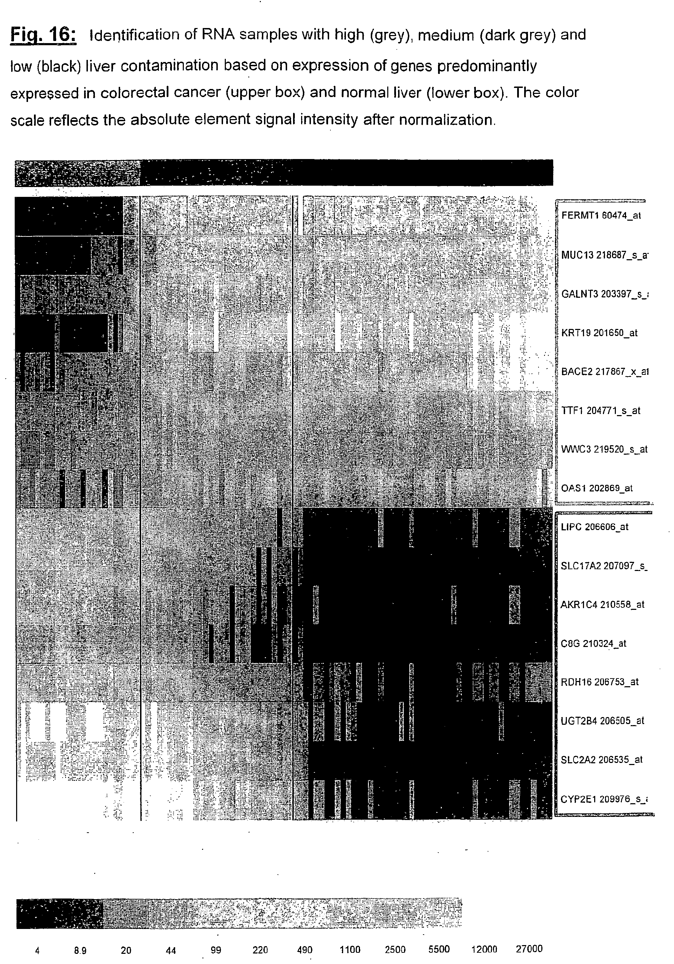

**Fig. 16****:** Identification of RNA samples with high (green), medium (red) and low (black) liver contamination based on expression of genes predominantly expressed in colorectal cancer (blue box) and normal liver (purple box). The color scale reflects the absolute element signal intensity after normalization.

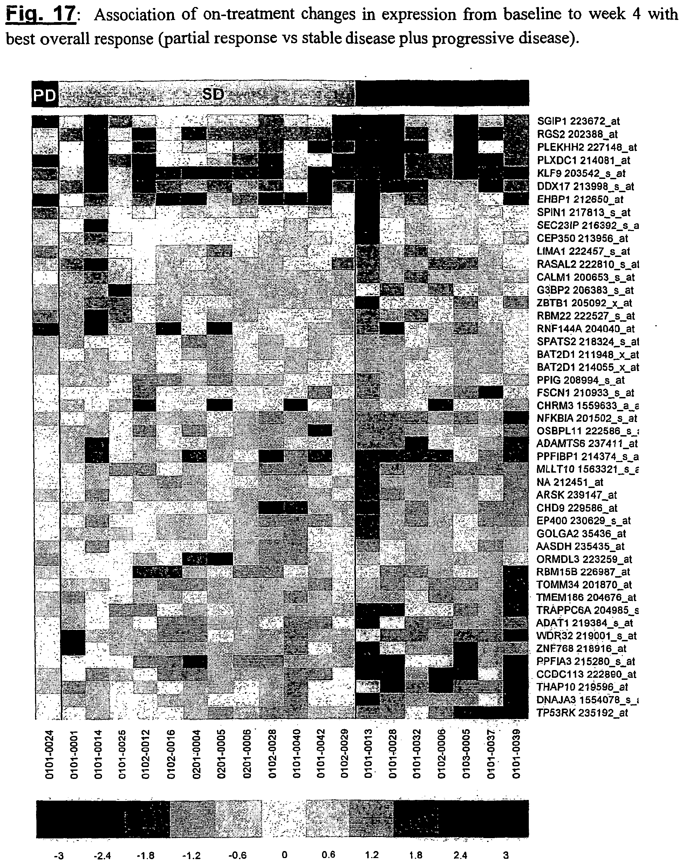

**Fig. 17****:** Association of on-treatment changes in expression from baseline to week 4 with best overall response (partial response vs stable disease plus progressive disease). Forty-seven probe sets with *P*<0.002 are shown. Gene names, followed by the element ID are given on the right of the image. The element intensity represents the log₂ ratio of gene expression at week 4 over gene expression at baseline.
   Abbreviations; PD, progressive disease; SD, stable disease; PR, partial response.

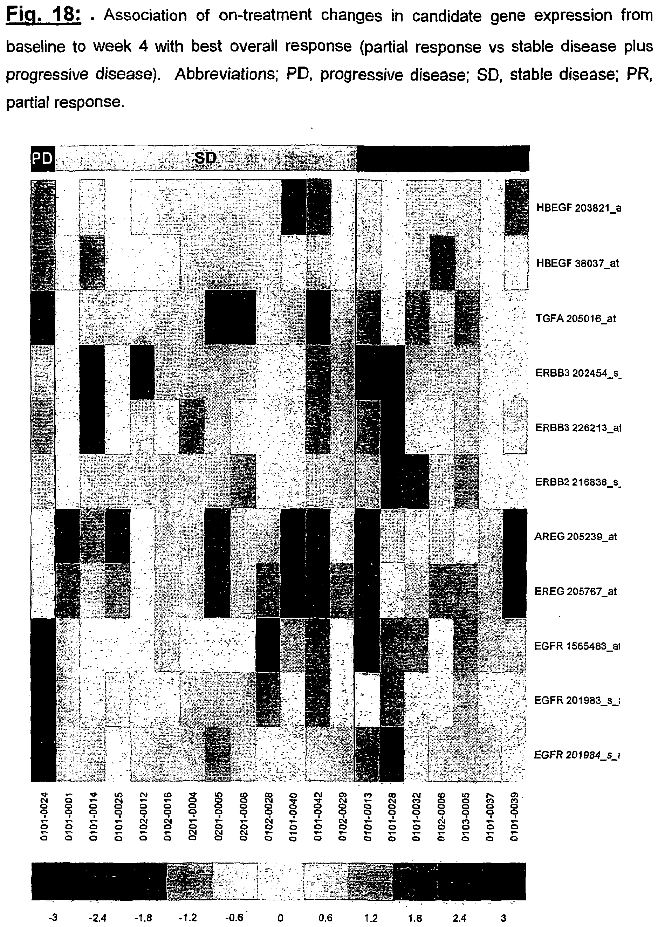

**Fig. 18****:** Association of on-treatment changes in candidate gene expression from baseline to week 4 with best overall response (partial response vs stable disease plus progressive disease). Gene names, followed by the element ID are given on the right of the image. The element intensity represents the log₂ ratio of gene expression at week 4 over gene expression at baseline.
   Abbreviations; PD, progressive disease; SD, stable disease; PR, partial response.

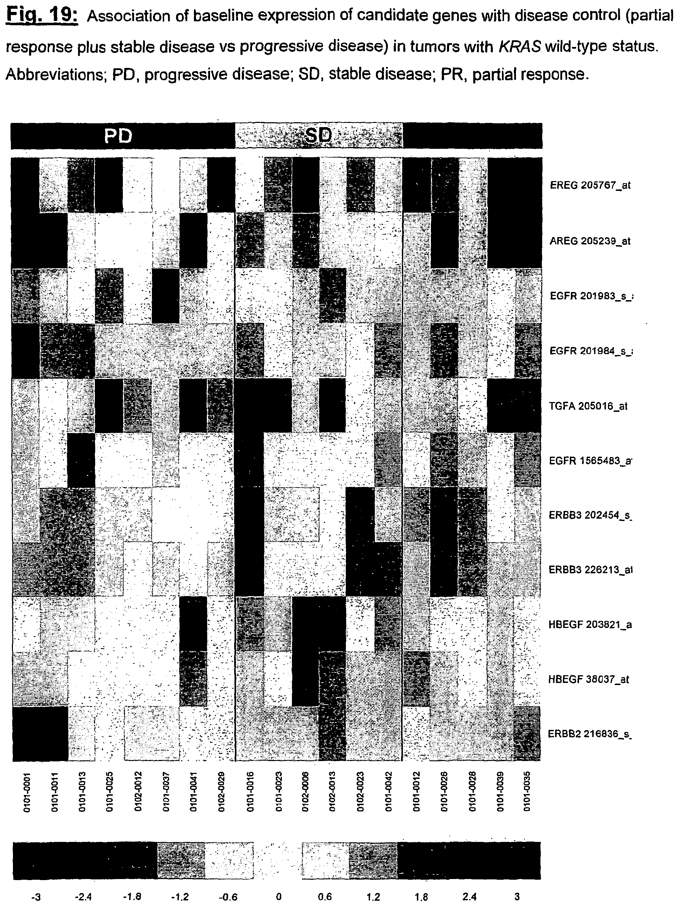

**Fig. 19****:** Association of baseline expression of candidate genes with disease control (partial response plus stable disease vs progressive disease) in tumors with *KRAS* wild-type status. Gene names, followed by the element ID are given on the right of the image. The intensity scale reflects the log₂ ratio for each element, relative to the mean for each probe set across all samples Abbreviations; PD, progressive disease; SD, stable disease; PR, partial response.

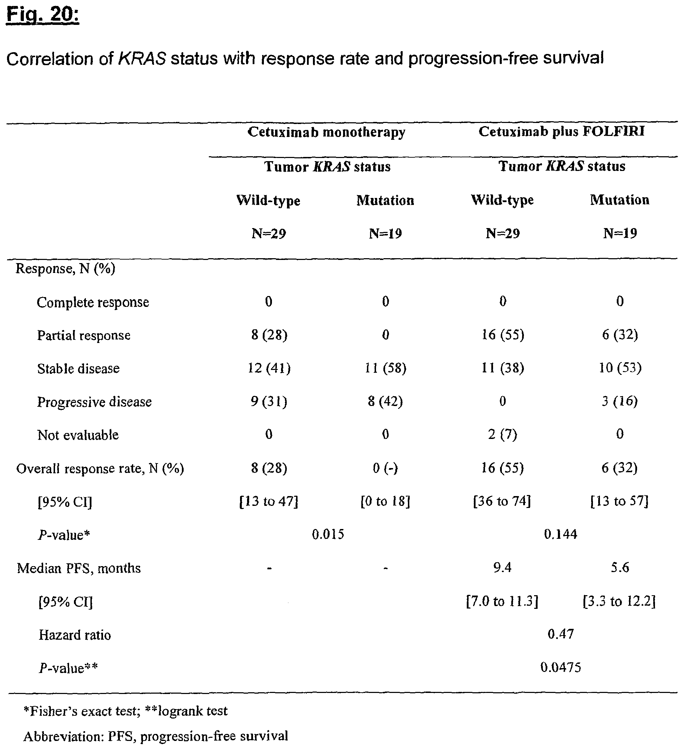

**Fig. 20****:** Correlation of *KRAS* status with response rate and progression-free survival in mCRC patients treated with cetuximab (Erbitux)

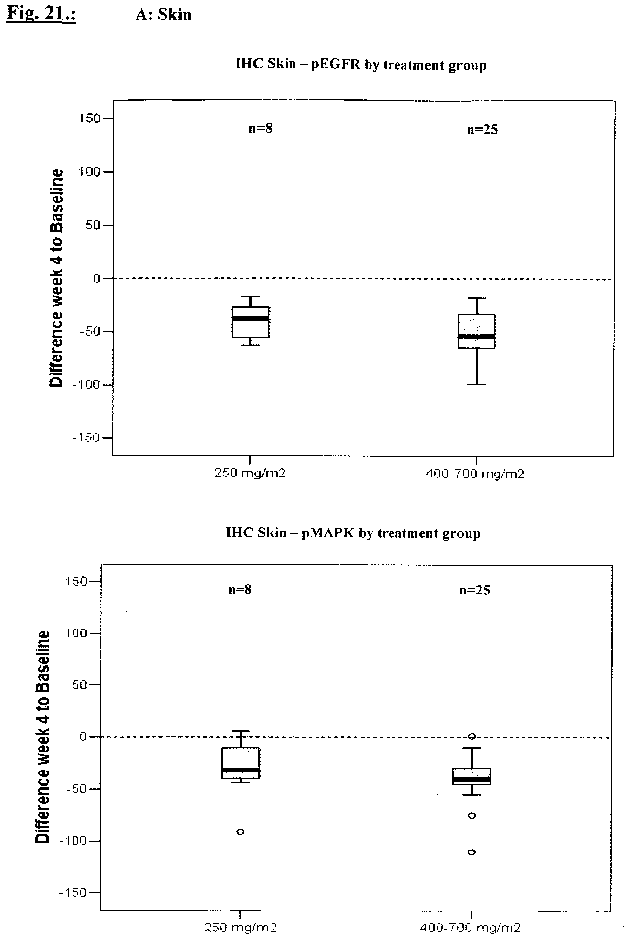

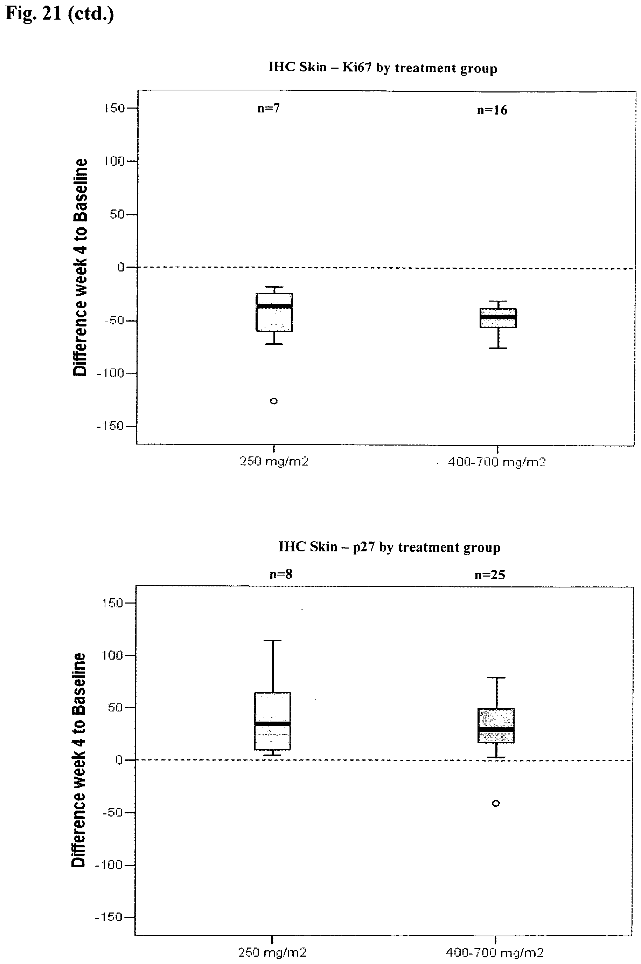

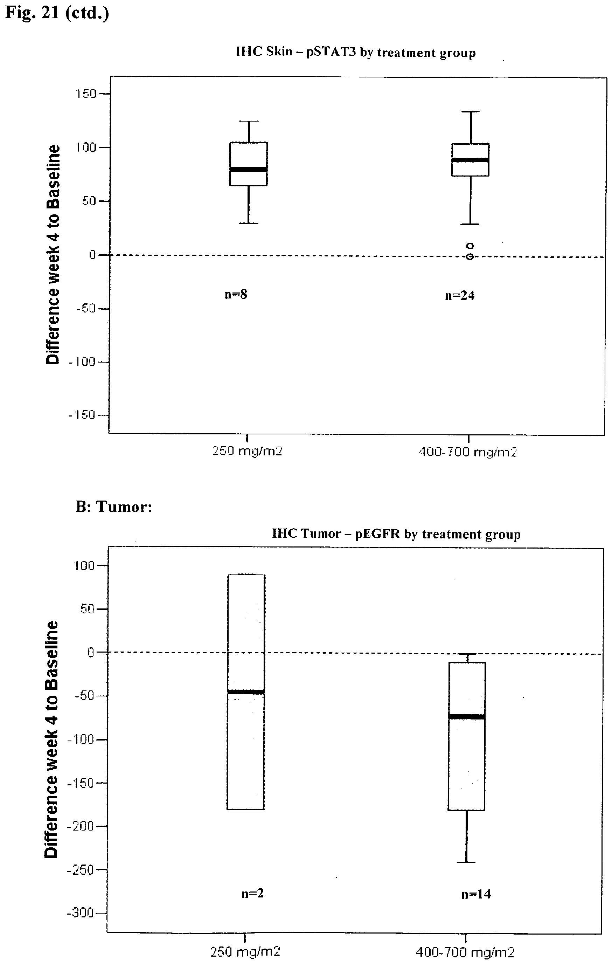

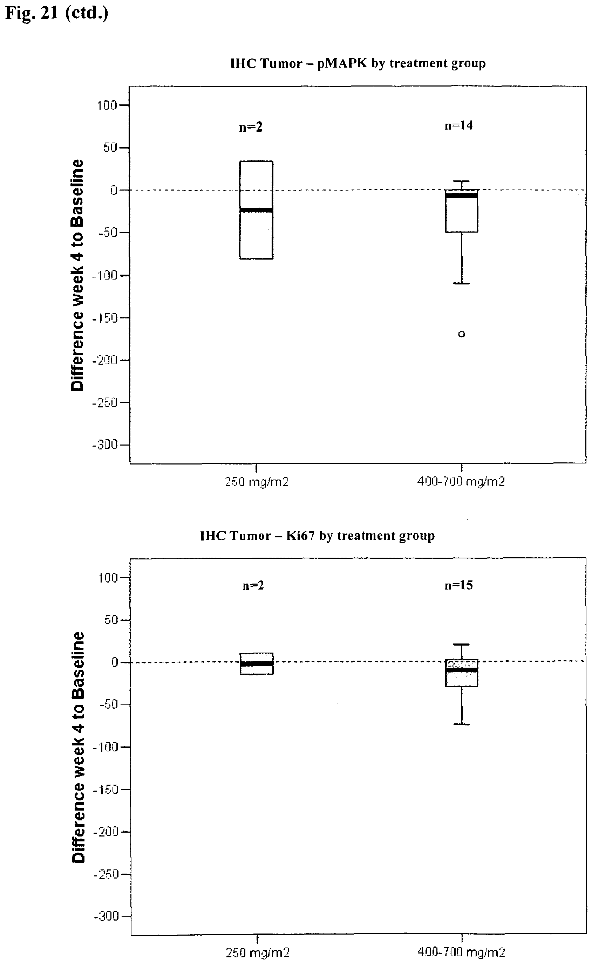

**Fig 21****.** Immunohistochemical analysis of the expression of selected EGFR signaling pathway associated markers in skin (A) and tumor (B) samples: changes between paired week4/baseline samples.

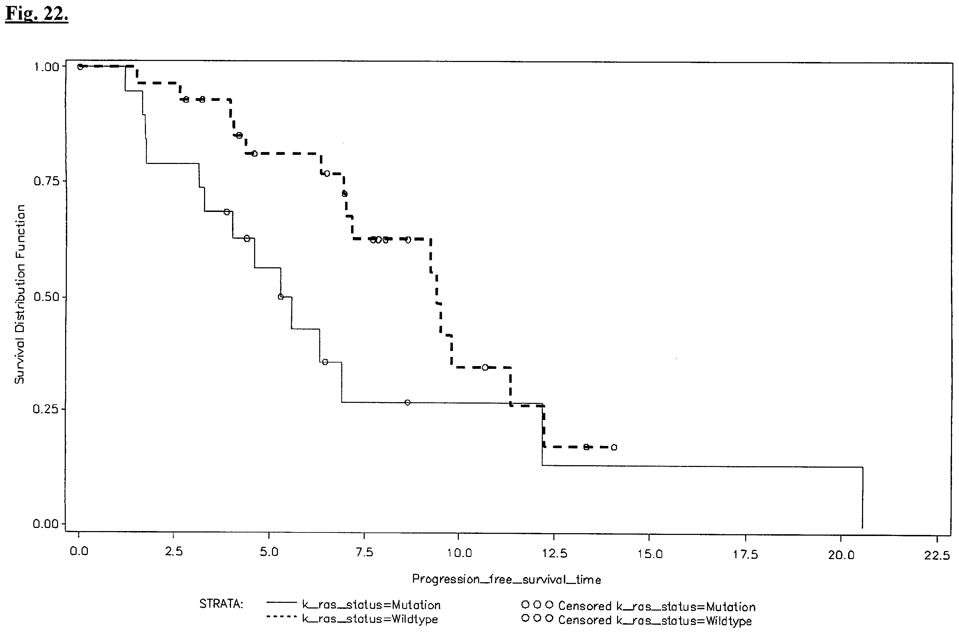

**Fig. 22****.** Proportion of patients free of disease progression versus progression-free survival time in months according to *KRAS* tumor mutation status.

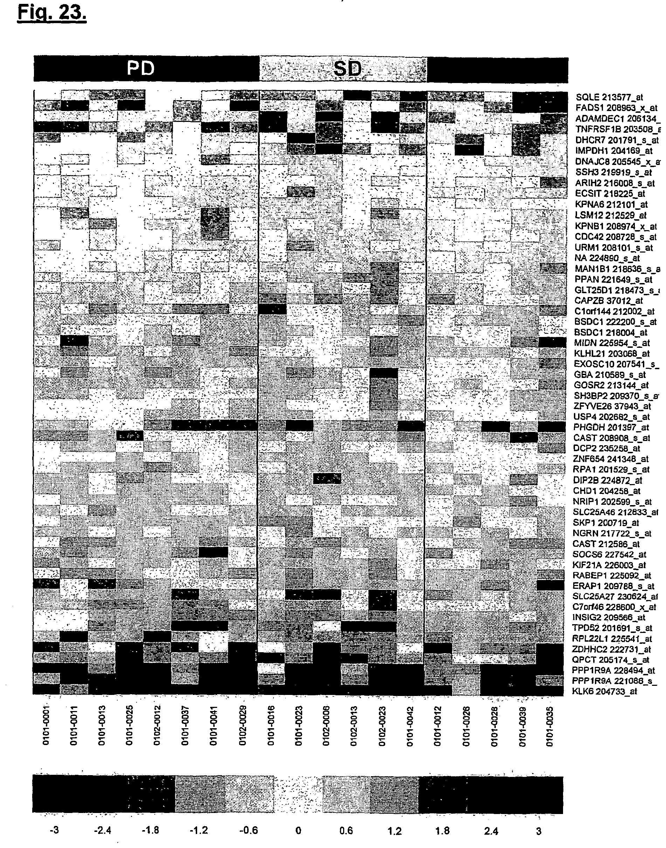

**Fig. 23****.** Association of baseline gene expression data with disease control (partial response plus stable disease vs progressive disease) at week 6 in patients with *KRAS* wild-type tumors. 57 probe sets with *P*<.002 are shown. Gene names, followed by the element ID are given on the right of the image. The color scale reflects the log₂ ratio for each element, relative to the mean for each probe set across all samples.
   Abbreviations; PD, progressive disease; SD, stable disease; PR, partial response.

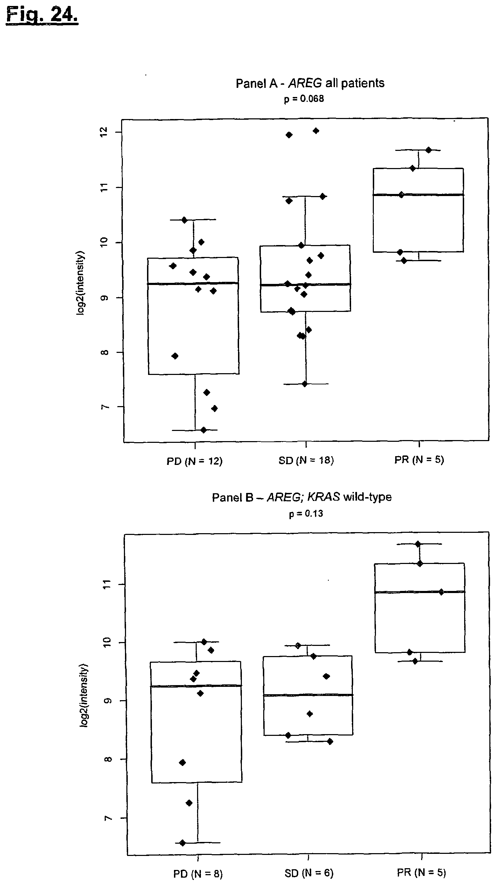

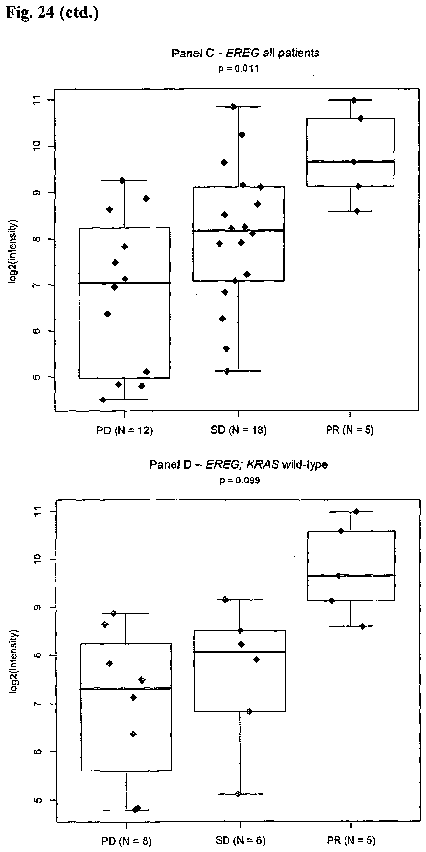

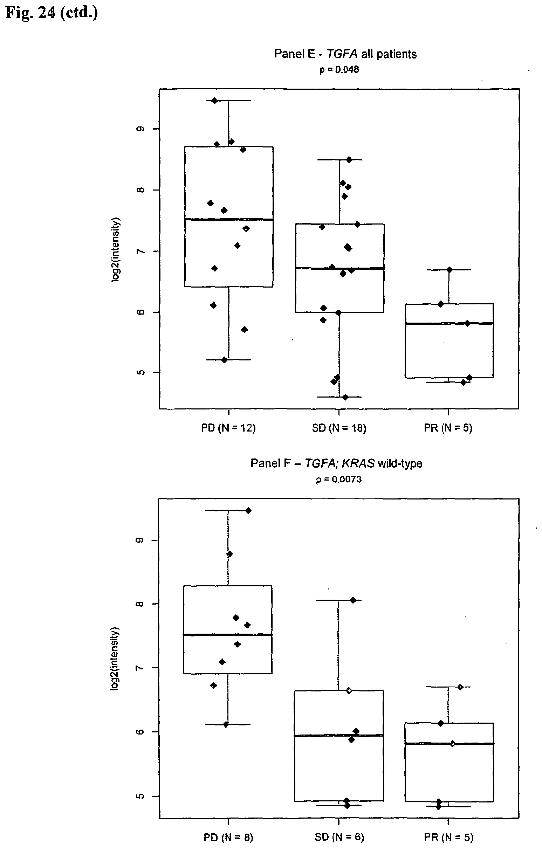

**Fig.24**. *AREG* (element 205239_at), *EREG* (element 205767_at) and *TGFA* (element 205016_at) expression levels in baseline samples according to response at week 6 in all patients (Panel A, C, and E, respectively) and in patients whose tumors were wild-type for *KRAS* (Panels B, D, and F, respectively). *P*-values refer to the association with disease control (partial response, PR and stable disease, SD versus progressive disease, PD).

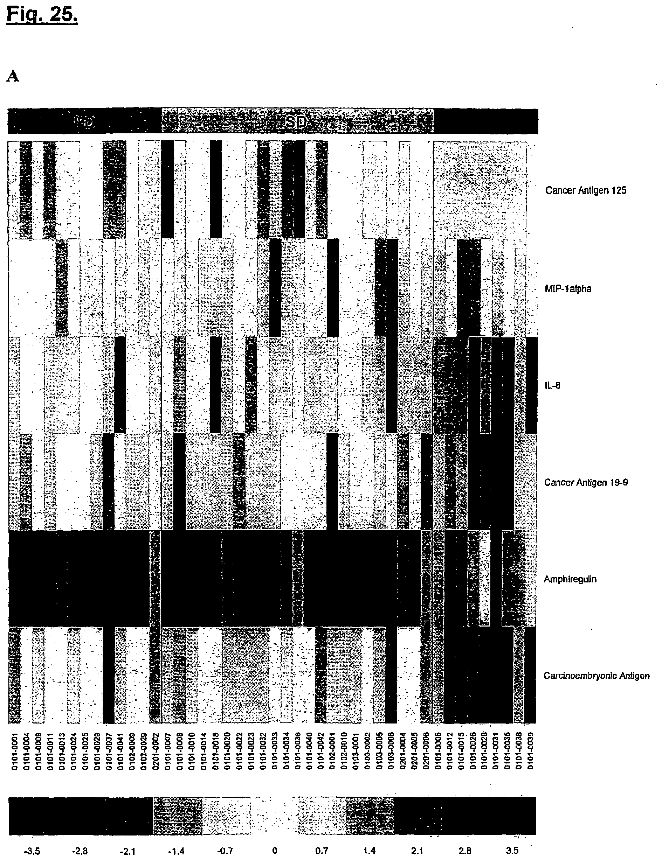

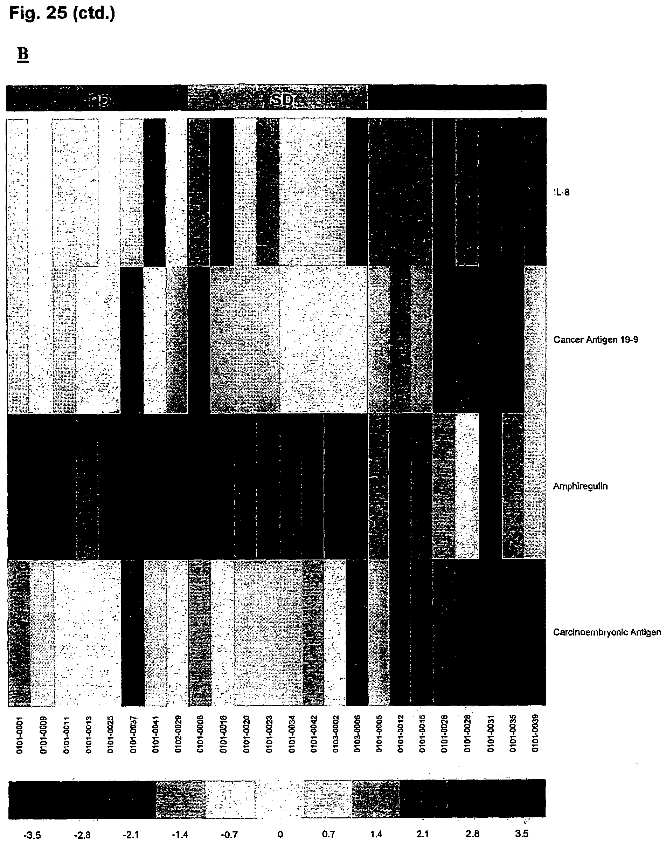

**Fig. 25****.** Association of on-treatment changes in plasma protein concentrations from baseline to week 4 with response at week 6 (partial response, PR vs stable disease, SD plus progressive disease, PD) among 45 patients in the intention to treat (ITT) population (Panel A) and among 24 ITT patients with *KRAS* wild-type tumors (Panel B). All proteins with P<.01 are shown. The element intensity represents the log₂ ratio of protein concentration at week 4 over protein concentration at baseline.

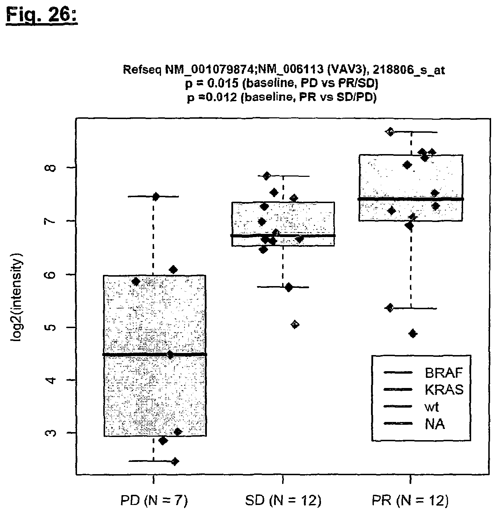

**Fig. 26****:** Boxplots showing the association of VAV3 with response. PD: progressive disease, PR: partial response, SD: stable disease. Green dots: Patients with KRAS and BRAF wild-type tumors, red dots: patients with KRAS mutations, black dots: patients with BRAF mutations, blue dots: mutation status unknown. P-values are based on Welch t-tests.

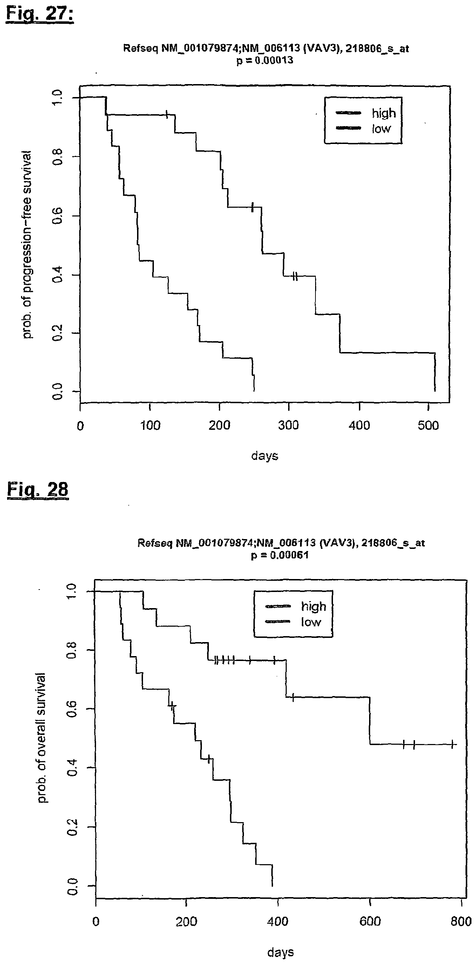

**Fig. 27****:** Kaplan-Meier plot showing estimated progression-free survival distribution functions stratified by VAV3 expression. Patients have been classified as high or low VAV3 expressors, depending on whether their baseline VAV3 expression levels were above or below the median level across patients, respectively. The p-value is derived from a Cox proportional hazards model.
**Fig. 28****:** Kaplan-Meier plot showing estimated overall survival distribution functions stratified by VAV3 expression. Patients have been classified as high or low VAV3 expressors, depending on whether their baseline VAV3 expression levels were above or below the median level across patients, respectively. The p-value is derived from a Cox proportional hazards model.

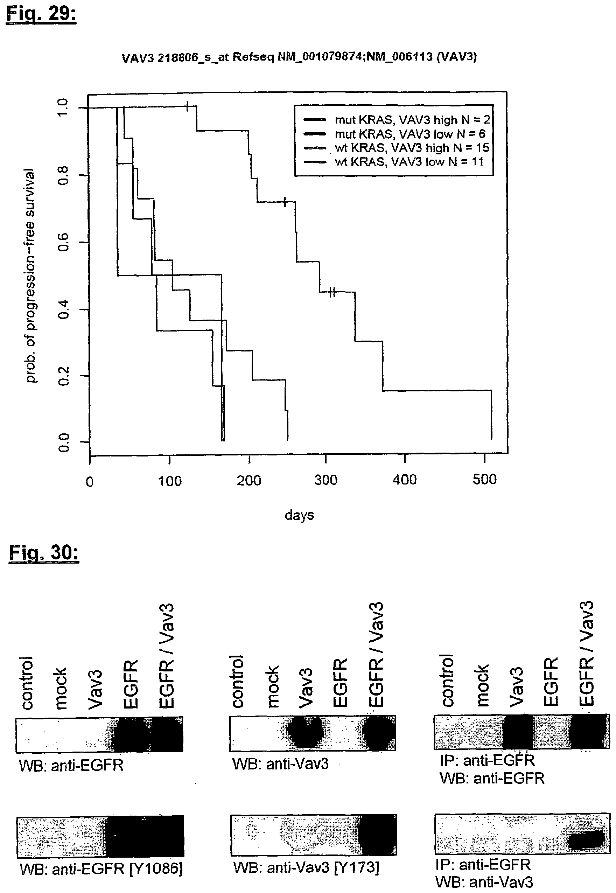

**Fig. 29****:** Kaplan-Meier plot showing estimated progression-free survival distribution functions stratified by VAV3 expression and KRAS mutation status. Patients have been grouped into four strata, representing all possible combinations of KRAS mutation status and baseline VAV3 expression (above or below the median).
**Fig. 30****:** Vav3 interacts with activated EGFR. After transfection of HEK 293 cells with VAV3 and EGFR alone or in combination, cells were lysed and subjected to immunoprecipitation (IP) and Western blotting (WB).

### DETAILED DESCRIPTION OF THE INVENTION

The EGFR-targeting immunoglobulin (Ig)G1 monoclonal antibody, cetuximab, was the first monoclonal antibody to be approved for the treatment of a solid tumor.

Intensive research on usable biomarkers to predict cetuximab response has been conducted to identify those patients who will benefit most significantly from cetuximab treatment. Tumor EGFR expression as assessed by immunohistochemistry has proved to be a disappointing biomarker for the efficacy of EGFR-targeted treatment in CRC. More promising data have been reported for mutations of the KRAS gene, which encodes a GDP/GTP-binding protein linking ligand-dependent receptor activation to intracellular pathways of the EGFR signaling cascade. Retrospective analyses of the KRAS mutation status in a multitude of clinical studies including two randomized studies of first-line treatment in metastatic CRC (mCRC), EMR 62202-047 and EMR 62202-013, as well as the randomized C0.17 study (investigating cetuximab monotherapy in patients with mCRC who had failed prior chemotherapy) have demonstrated that KRAS codon 12/13 mutation status is predictive for cetuximab activity in CRC. Tumor responses are predominantly seen in subgroups of patients whose tumors were wild-type for KRAS and patients carrying a KRAS codon 12/13 mutation do not benefit from cetuximab therapy. The mutation status of KRAS therefore appears to be a powerful predictive biomarker for cetuximab activity in CRC, allowing the exclusion from treatment of a subpopulation unlikely to derive a significant benefit.

Yet, not all of the about 60% of CRC patients with KRAS wild-type tumors do benefit from cetuximab treatment. About 40% of patients with KRAS wild-type tumors do not respond to cetuximab treatment and a substantial fraction of these patients progresses early and has a short overall survival.

Therefore, there is a need for the identification and use of further biomarkers that can be used in addition to the KRAS mutation status to better predict the clinical outcome of cetuximab treatment in CRC patients. It is further a need to identify of biomarkers that allow a better prediction of cetuximab efficacy in the treatment of CRC in addition to KRAS mutation status.

Microarray analysis of fresh frozen liver metastasis biopsies was performed in the two cetuximab CRC studies EMR 62202-502 and EMR 62202-045 to identify genes whose expression is associated with response, progression-free survival or overall survival in the general patient population or in patients with KRAS wild-type tumors. The expression of these genes can be used as a predictive biomarker for efficacy of cetuximab treatment in CRC and to better identify those patients who will derive most benefit from cetuximab treatment in CRC.

The expression of the above described genes are used as biomarkers for predicting efficacy of cetuximab and other anti-EGFR directed therapeutic antibodies in patients with CRC and facilitate treatment decisions in the clinic, i.e. if a patient will receive cetuximab or other anti-EGFR directed therapeutic antibodies or not. The application in clinical practice is:
1. Analysis of mRNA expression of these genes from formalin-fixed paraffin embedded (FFPE) or fresh tumor biopsies (the latter have to be either directly frozen in liquid nitrogen or treated with RNA-later to conserve RNA integrity). The biopsies can be obtained from primary tumor or metatasis. Analysis of mRNA expression is performed by PCR-based methods (e.g. real-time PCR, qPCR) by using gene specific primers to amplify the gene of interest or by hybridization of the mRNA of the gene of interest to gene specific, immobilized hybridization probes on gene arrays.
2. Analysis of protein expression of these genes from FFPE or fresh tumor biopsies (the latter have to be either directly frozen in liquid nitrogen or treated with RNA-later to conserve RNA integrity). The biopsies can be obtained from primary tumor or metatasis. Analysis of protein expression includes methods such as immunohistochemistry, enzyme-linked immunosorbent assay (ELISA), Luminex, blotting and detection of proteins on membranes, mass spectrometry.

For soluble proteins: Analysis of protein expression from plasma or serum comprising methodologies such as ELISA, Luminex, mass spectrometry.

For establishing a diagnostic assay for clinical practice, the expression levels of the candidate gene(s) or protein(s) need to be normalized against the expression of another gene or protein (or a combination of genes and proteins) that is (are) assessed from the same biopsy with the same assay method. These "normalization" genes or proteins can comprise cellular house-keeping genes that are known to display very low variation from patient to patient. Alternatively, the ratio of the expression level of a gene or protein (or a combination of genes or proteins) from the "good-prognosis" (or whatever terminology we will use in the end (e.g. "sensitivity") group and the expression level of a gene or protein (or a combination of genes or proteins) from the "bad-prognosis" (or e.g. "resistance") group can be determined. The latter approach offers the advantage of using only expression levels of genes or proteins that are directly linked to the efficacy of the anti-EGFR therapy. This approach results in a high dynamic range and is independent of suitable house-keeping genes or proteins.

Prior to implementation of the diagnostic assays a threshold has to be defined which is the ratio of expression levels of the applied markers (as described above) that has to be achieved in order to trigger a positive decision for treating a patient with the respective anti-EGFR therapy. This threshold should discriminate between patients who benefit and patients who do not benefit from the anti-EGFR treatment in the best possible way. Such a threshold has to be derived from a "training-set" of tumor samples from patients treated with the anti-EGFR therapy. Then the threshold has to be prospectively validated in a different set of tumor samples from a sufficient number of patients to prove its ability to select patients who will derive most benefit or to exclude patients who will not benefit from treatment.

In two independent clinical studies (EMR 62202-502 and EMR 62202-045) for treatment of CRC patients with cetuximab the expression of the genes described above and in the following was found to be associated with response and/or progression-free survival and/or overall survival.
- EGFR gene amplification may predict favorable outcome to anti-EGFR therapy
- Subsequent studies found a lower incidence of EGFR gene amplification
- Methodology/comparability issues
- K-Ras mutations "override" benefit in patients with EGFR gene amplification
- Skin-rash is up to now probably the best predictable biomarker for Erbitux activity (mCRC, NSCLC)
- PhIII study FLEX of Erbitux: early onset of skin rash (first 21 days) associated with prolonged over-all survival (OS)
- BRAF mutations found in 5% of patients
- BRAF and KRAS mutations were mutually exclusive
- BRAF mutation appears to be rather a bad prognostic marker than a predictive marker for Erbitux efficacy.
- AREG and EREG expression in mCRC is independent of the KRAS mutation status
- Additional predictive power by combining AREG and EREG expression status with KRAS mutation status

Cetuximab treatment according to the experiments of the invention was associated with substantial downregulation of p-EGFR, p-MAPK and proliferation and substantial upregulation of p27^{Kip1} and p-STAT3 levels in basal keratinocytes. No marked difference in these effects was noted for the different schedules of administration and dose levels. In the cetuximab monotherapy phase, responses were achieved only in patients whose tumors were wild-type for *KRAS* (8/29 vs 0/19 for *KRAS* mutant tumors; *P*=.015). Progression-free survival was longer for patients with *KRAS* wild-type compared with *KRAS* mutant tumors (logrank, *P*=.048). Genomics/proteomics analyses identified candidate markers associated with response.

This translational study conducted in a phase I dose-escalation trial of cetuximab monotherapy constitutes to our knowledge the first attempt to use pharmacogenomic and pharmacoproteomic analyses to identify predictive pretreatment biomarkers for cetuximab-responsive mCRC in the first-line setting.

The clinical study (to be reported elsewhere) demonstrated that cetuximab can be safely administered as first-line therapy to patients with mCRC every second week at doses of 400-700 mg/m². The MTD was not reached at the highest dose level, and there were no marked differences in the incidence or severity of adverse events or the activity of cetuximab at different dose levels. Using the skin to measure target impact, the IHC data in the pharmacodynamic biomarker evaluation showed consistent inhibition of signaling proteins within the EGFR pathway across the dose-escalation groups. These data provide a biological rationale supporting the functional equivalence of weekly and every second week dosing regimens.

Retrospective analyses of single arm and randomized mCRC studies have confirmed that the mutation status in tumors of *KRAS* at codons 12 and 13 is a strong predictor of cetuximab activity, with treatment benefit tightly linked to wild-type status.^{4,7-13} The current study addressed for the first time the influence of *KRAS* mutation status on cetuximab monotherapy in the first-line treatment of mCRC patients. Consistent with the data from previous series of chemorefractory patients treated with cetuximab as a single agent or in combination with chemotherapy,^{8,11,13} objective responses in the monotherapy part of the study were only observed in those patients with *KRAS* wild-type tumors (8 out of 29 patients, 28%), with no responses (0 out of 19) seen in patients with tumors that carried mutations in the gene (*P*=.015). The best overall response rates (including the responses after adding FOLFIRI) in patients with *KRAS* wild-type tumors (55%) and *KRAS* mutated tumors (32%) were comparable to results obtained in the CRYSTAL and OPUS studies, where cetuximab was combined as first-line treatment respectively with FOLFIRI and FOLFOX.^{4,12} The observed responses in mCRC patients with *KRAS* mutated tumors receiving cetuximab in combination with chemotherapy as first-line treatment in the current study are most likely attributable to the effect of chemotherapy. Overall PFS was significantly longer for patients whose tumors were wild-type for *KRAS,* demonstrating the clinical significance of *KRAS* tumor mutation status as a predictive biomarker in relation to cetuximab treatment.

A subset of patients with *KRAS* wild-type tumors do not appear to benefit from cetuximab treatment. It may therefore be possible to identify further predictive biomarkers which will facilitate the more accurate tailoring of treatment to those patients who will respond to cetuximab. Recently, the negative predictive value of *BRAF*¹⁸ and *PI3K*^{19,20} mutations as well as PTEN deregulation¹⁹⁻²¹ has been preliminarily described. Other molecular markers which have been putatively associated with the clinical activity of cetuximab include tumor expression levels of *VEGF, IL8, EGFR,* and *PTGS2* (*COX2*);²² circulating levels of VEGF during treatment;²³ constitutional polymorphisms of *PTGS2* and *EGFR*²⁴ and *TP53* tumor mutation status.²⁵

For a range of anticancer agents high-throughput genomics technologies are increasingly being utilized in the search for predictive biomarkers.²⁶⁻²⁹ In the case of cetuximab, high-level expression of genes encoding the EGFR ligands AREG (amphiregulin) and EREG (epiregulin) in tumors has been shown to be associated with clinical activity in mCRC patients, both by microarray^{10,30} (unselected population and a population with *KRAS* wild-type tumors) and quantitative reverse transcriptase-PCR³¹ (patients receiving cetuximab plus irinotecan) approaches. Similarly, in the current first-line study, *AREG* and *EREG* expression appeared to be elevated in tumors of patients without disease progression, in both the total population and the *KRAS* wild-type tumor subgroup. In contrast, *TGFa* (encoding TGF-α), showed lower levels of expression in patients without disease progression. The global gene expression analysis of *KRAS* wild-type tumors identified 57 genes putatively associated with disease control at week 6 (*P*<.002). Among these candidates, six genes (*TNFRSF1B*, *DNAJC8, ECSIT, GOSR2, PPP1R9A,* and *KLK6*) were found to have a False Discovery Rate <0.1. The value of these putative biomarkers for improving prediction of cetuximab efficacy in *KRAS* wild-type mCRC needs further exploration.

Luminex analysis of plasma proteins revealed a strong increase in the levels of amphiregulin and TGF-α during cetuximab monotherapy treatment, a trend that was also seen for EGF. The upregulation of these EGFR ligands might be a compensating reaction to EGFR inhibition. Interestingly, the increase in amphiregulin levels was significantly lower in patients who responded to cetuximab treatment. A significant decrease of carcinoembryonic antigen and the cancer antigens 125 and 19-9 was observed under cetuximab monotherapy in responders. Remarkably also, the decrease in IL-8 levels was significantly associated with response in all tumors as well as in *KRAS* wild-type tumors. IL-8 is a pro-inflammatory cytokine that promotes proliferation and survival of tumor cells and has profound effects on the tumor microenvironment.³² IL-8 seems to be a predictive biomarker for cetuximab efficacy.

Furthermore according to the invention a direct interaction of EGFR and VAV3 could be detected when EGFR and VAV3 were expressed in HEK 293 cells. This indicates a direct and outstanding role for VAV3 in EGFR signaling and a direct link between observed high VAV3 expression levels and modulation of the activity of anti-EGFR therapy with cetuximab.

The invention shows for the first time that treatment with anti-EGFR antibodies, preferably cetuximab (every second week and weekly administration) as a single agent in a first-line setting benefits mCRC patients who have *KRAS* wild-type tumors. In addition, the global gene expression analyses of this early-phase study have generated a number of interesting results regarding the expression of certain genes and the clinical activity of cetuximab. These observations enable validation on larger patient series using different methodologies. The results of these studies provide a rational foundation for optimizing treatment in patients suffering from different cancers, especially CRC or mCRC with cetuximab or anti-EGFR antibodies being similarly active.

### Immunohistochemical Analysis of EGFR Pathway Components

Evaluable paired baseline/week 4 skin biopsies to analyze pharmacodynamic changes of the assessed markers were available from up to 35 patients. Substantial downregulation of p-EGFR, p-MAPK and proliferation (as assessed by Ki67 staining) was observed in the 4-week compared with baseline samples. In parallel, a substantial upregulation of p27^{Kip1} and p-STAT3 was observed . In the analysis of different schedules of administration and dose levels, no relevant differences in relation to changes in the levels of these markers between groups of patients were present for baseline to on-treatment timepoints (Fig. 21A).

Evaluable paired baseline/week 4 tumor biopsies were available from up to 17 patients. Reduction in proliferation and a profound downregulation of p-EGFR and p-MAPK were observed in tumor cells after therapy (Fig. 21B). However, p27^{Kip1}, p-STAT3 and p-AKT levels were not markedly modified by cetuximab treatment (data not shown). The small number of available paired tumor biopsies did not allow a comparison of changes in biomarker levels with dose groups and response variables.

### KRAS Mutation Analysis

*KRAS* codon 12 or 13 mutations were detected in 19/48 (40%) patient samples (G12V, 9 patients; G13D, 5 patients; G12D, 4 patients; G12A, 1 patient). In the cetuximab monotherapy phase, there were eight partial responses (PRs) among the 48 patients. All were in patients whose tumors were wild-type for *KRAS* (8/29; 28%). No responses were reported in the 19 patients whose tumors carried *KRAS* mutations (P=.015) (Table 1). In the study overall (monotherapy and combination therapy phases), responses were seen in 16/29 (55%) of patients with *KRAS* wild-type and 6/19 (32%) with *KRAS* mutant tumors (*P*=.144). PFS was significantly longer in patients whose tumors were wild-type for *KRAS*, compared to those whose tumors carried mutations (Fig. 22; median 9.4 vs 5.6 months, hazard ratio 0.47; logrank *P*=.048).

### Microarray Analysis of Gene Expression

A total of 106 tumor-derived samples from baseline and 4-week timepoints were hybridized to Affymetrix GeneChip HG-U133 Plus 2.0 arrays. Four arrays were excluded from further analysis on the basis of general quality control parameters and 24 samples were excluded due to presence of normal liver tissue contamination (Fig. 16; supplemental material) and were not further analyzed. After the exclusion of duplicates, 62 array data sets from 42 ITT patients (36 baseline, 26 week 4: 20 pairs) remained for analysis.

For the analyzed tumor samples, the data from the 54,675 probe sets was pre-filtered on the basis of variance, signal intensity and probe set annotation (see Supplementary Methods). This process restricted the tumor expression analysis to 15,230 probe sets, representing 10,538 genes. In global comparisons of baseline pre-filtered data according to response: progressive disease (PD; n=12) versus disease control at 6 weeks (n=23; 1 patient not evaluable) and for best overall response: PD and stable disease (SD) (n=19) versus PR (n=14; 3 patients not evaluable), the distribution of *P* values (data not shown) was essentially as expected by chance, suggesting that a gene expression profile predictive of response had not been identified for the global population. However, restricting the analysis to *KRAS* wild-type tumors (8 patients with PD versus 11 patients with disease control), 57 probe sets with expression patterns putatively associated with disease control at week 6 were identified (P<.002; Fig 23). Imposing a False Discovery Rate (FDR) threshold of 0.1 (for FDR definition see Supplementary Methods section), six genes were found to be significantly associated with disease control (*TNFRSF1B*, *P*=6.90E-07; *DNAJC8, P*=1.60E-06; *ECSIT, P*=6.80E-06; *GOSR2, P*=3.90E-05 with higher expression in patients showing disease control and *PPP1R9A*, *P*=8.90E-07 and *KLK6, P*=3.00E-05 with higher expression in patients with PD).

On treatment changes associated with response were examined using data from patients with available samples from the baseline and the week 4 timepoints. No expression changes were identified that appeared to be tightly associated with disease control at week 6 (n=12) compared with PD (n=8). Considering the combination with chemoptherapy, the comparison of profiles for PR (n=7) versus SD/PD (n=13), revealed 47 probe sets showing differences in on-treatment changes (*P*<.002, moderated t-test, see Fig. 17).

In patients with *KRAS* wild-type tumors, as well as in the complete set of analyzed patients, baseline *EREG* (epiregulin) and *AREG* (amphiregulin) expression levels were higher in those tumors responding to cetuximab (Fig. 24; Fig. 18). These findings are in line with results reported by Khambata-Ford et al.¹⁰ Interestingly, *TGFA* (TGF-□) demonstrated a reciprocal expression pattern (Fig. 24; Fig. 18). Among other genes known to be directly or indirectly involved in EGFR signaling such as ERBB receptors and ligands *ERBB3* (*HER3*) and *ERBB2 (HER2)* showed a trend for stronger downregulation in tumors with a PR as best overall response (Fig. 19).

### Plasma Proteomics

The concentrations of 97 different proteins were analyzed in plasma samples using Luminex technology. The protein panel included EGFR ligands, other growth factors, interleukins and a variety of other candidate proteins. During the cetuximab monotherapy phase, the decrease in plasma levels of interleukin (IL)-8, macrophage inflammatory protein (MIP)-1□ as well as the tumor markers carcinoembryonic antigen, cancer antigens 125 and 19-9 between baseline and week 4 was significantly associated (*P*<.01) with response at week 6 (Fig. 25A). The general strong increase in plasma concentrations of amphiregulin was significantly (*P*<.01) weaker in patients with partial response to cetuximab monotherapy (Fig. 25). The association with response at week 6 was also found for carcinoembryonic antigen, cancer antigen 19-9, IL-8 and amphiregulin when the analysis was restricted to patients with *KRAS* wild-type tumors (data from 24 patients, Fig. 25B). Furthermore a general increase (independent of response) of TGF-α and EGF levels and a decrease of soluble EGFR were observed in plasma during the first 4 weeks of cetuximab monotherapy treatment.

Among the investigated genes and candidates showing an association between expression levels and success of therapy in patients with metastatic colorectal cancer (mCRC) receiving cetuximab, VAV3 is of particular interest. In study EMR 62202-502 high tumoral VAV3 mRNA expression levels were not only strongly associated with better response to cetuximab in combination with irinotecan (Figure 26) but also with progression free survival (PFS) (Figure 27) and overall survival (OS) (Figure 28). Furthermore, high tumoral VAV3 expression levels were found to be particularly associated with response and prolonged PFS in patients with KRAS wild-type tumors (see Figure 1 and Figure 29). Therefore, VAV3 expression appears to be a good biomarker candidate for predicting clinical outcome of cetuximab therapy in CRC in patients with KRAS wild-type tumors which would help to further optimize selection of patients deriving most benefit from cetuximab therapy.

Interestingly, a direct interaction of EGFR and VAV3 could be detected when EGFR and VAV3 were expressed in HEK 293 cells (Figure 30). This suggests a direct role for VAV3 in EGFR signaling and a direct link between VAV3 expression levels and modulation of the activity of anti-EGFR therapy.

### Examples

### Clinical studies:

EMR 62202-502: This randomized study investigated cetuximab dose-escalation in patients (pts) with EGFR-expressing mCRC failing irinotecan-including therapy. Pts were randomized 22 days after starting cetuximab (400 mg/m2 initial dose then 250 mg/m2/week [w]) with I (180 mg/m2 q 2 w) if they had not experienced >grade (G) 1 skin reaction, any other >G 2 cetuximab-related adverse event and were tolerant to I. Randomization was to standard cetuximab dose (Arm A; 250 mg/m2/w) or dose-escalation (Arm B; cetuximab dose increased by 50 mg/m2 q 2 w, until >G 2 toxicity, tumor response or dose = 500 mg/m2). Pts not randomized (Arm C) continued on standard cetuximab dose. Primary endpoint was to compare in skin and tumor biopsies, taken before and during treatment, the effects of dose-escalation on EGFR and downstream signalling markers with those of the standard cetuximab regimen. Secondary endpoints were PK, efficacy, safety, tolerability, biomarker analyses on tumor biopsies and plasma samples. The KRAS mutation status was analyzed from tumor biopsies.

EMR 62202-045: This study examined the safety and pharmacokinetics of every second week administration of cetuximab in patients with metastatic colorectal cancer. Secondary objectives included a pharmacodynamic biomarker analysis. Patients received cetuximab monotherapy for 6 weeks, followed by cetuximab plus FOLFIRI until disease progression. Patients in the control arm received cetuximab as a 400 mg/m2 initial dose then 250 mg/m2/week and in the dose-escalation arms, at 400-700 mg/m2, every second week. The KRAS mutation status was analyzed from tumor biopsies.

### Tumor material for Gene expression (Microarray) analysis:

EMR 62202-502: Tumor material was taken by open surgery, endoscopy or core/fine needle biopsy at baseline (pre-treatment), at day 22 and if possible, at disease progression of patients in the dose-escalation arm (Arm B). The samples were snap-frozen in liquid nitrogen.

EMR 62202-045: Tumor material was taken by open surgery, endoscopy or core/fine needle biopsy at baseline, at week 4 and if possible, at disease progression. The samples were snap-frozen in liquid nitrogen.

### RNA Expression Profiling

Experimental procedures related to the microarray analysis are detailed in the Supplementary Methods section. Briefly, snap-frozen tumor biopsies were homogenized and total RNA was extracted using an RNeasy Micro Kit® (Qiagen, Hilden). Biotinylated target cRNAs for the array hybridization experiments were prepared from all samples according to the Affymetrix Two-Cycle Eukaryotic Target labeling protocol. For each tumor analyzed, an initial 50 ng of total RNA was included in the first cDNA synthesis reaction of this cRNA amplification/labeling process. Labeled cRNA was subsequently hybridized to Affymetrix GeneChip HG-U133 Plus 2.0 gene expression arrays for 16 hours at 45 °C at 60 rpm. Following hybridization, arrays were stained on an Affymetrix Fluidics Station 450 and signal quantified using a GeneChip Scanner. Quality control and preprocessing of the raw expression data were carried out using the proprietary Affymetrix GCOS software and the Bioconductor package, affyPLM.

EMR 62202-502: After all quality control checks and pre-processing steps had been performed a total of 68 array data sets from 47 subjects of the intention to treat (ITT) population were eligible for further analysis. Baseline samples were available from 35 subjects.

EMR 62202-045: After all quality control checks and pre-processing steps had been performed a total of 62 array data sets from 42 ITT patients were eligible for further analysis. Baseline samples were available from 36 subjects.

Statistical analyses were conducted for all Affymetrix probe sets with reliable gene annotation that passed initial filters based on variability and signal intensity in at least one of the two studies (16414 Affymetrix probe sets representing 10785 genes).

Genes whose expression is associated with clinical response were identified with Welch t-test comparisons between responders and non-responders (EMR 62202-502), or between patients with disease control after six weeks of cetuximab monotherapy versus those with progressive disease (EMR 62202-045), respectively. Genes whose expression is associated with progression-free survival or with overall survival were identified using Cox proportional hazards regression (EMR 62202-502). These analyses were conducted for the complete sets of patients, as well as only for the patients with KRAS wildtype tumors.

A meta-analysis to identify response-associated genes across both studies was conducted using the products of single-study one-sided p-values as test statistic and deriving p-values from the null distribution of these products.

*P*-values in a range below 0.01 and 0.0001, specifically below 0.01, preferably 0.005, more preferably 0.002, most preferably 0.0005 or 0.0001 (from the meta-analysis for association with clinical response, and from the analysis of EMR 62202-502 for the association with progression-free and overall survival), were considered as statistically significant. This criterion was fulfilled for 200 Affymetrix probe sets representing 179 known genes in at least one of the comparisons.

### Patient Eligibility and Study Design

Eligibility criteria and study design have been reported in full in a separate manuscript. Briefly, the study was divided into two parts; a cetuximab monotherapy phase lasting 6 weeks and a combination therapy phase, during which patients received cetuximab, at the same dose/schedule as during the monotherapy phase, and the irinotecan-based schedule FOLFIRI. Patients were assigned sequentially to either the standard weekly schedule and dose of cetuximab (400 mg/m², followed by weekly doses of 250 mg/m²) or a cetuximab dose-escalation treatment on a bi-weekly schedule with different cohorts from 400 to 700 mg/m². Clinical response was reported after 6 weeks of cetuximab monotherapy and as best overall response (monotherapy and combination therapy phases).

### Collection and Storage of Patient Material

Skin biopsies were obtained at baseline and on day 26-28 (week 4). If skin rash was present, samples were taken from a rash-free area. The biopsy was immediately immersed into ≥20 times its volume of neutral-buffered formaldehyde solution at 4 °C, and held for 8-16 hours at room temperature. The fixed specimen was dehydrated to xylene using a graded ethanol series and embedded longitudinally in paraffin wax under vacuum at 60 °C. Tumor material was taken by open surgery, endoscopy or core/fine needle biopsy at baseline, at week 4 and if possible, at disease progression. One sample per timepoint was formalin fixed and paraffin embedded, as previously described^{13a} and three samples were snap-frozen in liquid nitrogen.

To provide normal DNA, 10 ml of whole blood was obtained from each patient at baseline and stored at -20 °C or lower until use. Plasma (2.5 ml) was collected for Luminex analysis at baseline and week 4, and stored at -80 °C.

### Immunohistochemistry

Immunohistochemical (IHC) analysis of formalin fixed paraffin embedded (FFPE) tissue was used to investigate the expression of the following proteins: EGFR, phospho(p)-EGFR, p-MAPK, Ki67 (MIB1), p27^{Kip1} (CDKN1B) and p-STAT3 (skin and tumor biopsies); HER2, p-HER2 and p-AKT (tumor biopsies). Immunohistochemistry analysis was performed as previously described.^{13a} Details of the antibodies and methods used are provided in the Supplementary Methods section.

### KRAS Mutation Analysis

FFPE patient-derived archival tumor tissue was available from 48 patients from the intention to treat (ITT) population. DNA was extracted and screened for the presence of *KRAS* codon 12 and 13 mutations using a polymerase chain reaction (PCR) clamping and melting curve technique adapted from Chen et al, 200414 (LightMix, k-ras Gly12, TIB MOLBIOL, Berlin, Germany), as previously described.¹²

### RNA Expression Profiling

Experimental procedures related to the microarray analysis are detailed in the Supplementary Methods section. Briefly, snap-frozen tumor biopsies were homogenized and total RNA was extracted using an RNeasy Micro Kit® (Qiagen, Hilden). Biotinylated target cRNAs for the array hybridization experiments were prepared from all samples according to the Affymetrix Two-Cycle Eukaryotic Target labeling protocol. For each tumor analyzed, an initial 50 ng of total RNA was included in the first cDNA synthesis reaction of this cRNA amplification/labeling process. Labeled cRNA was subsequently hybridized to Affymetrix GeneChip HG-U133 Plus 2.0 gene expression arrays for 16 hours at 45 °C at 60 rpm. Following hybridization, arrays were stained on an Affymetrix Fluidics Station 450 and signal quantified using a GeneChip Scanner. Quality control of the raw expression data was carried out using the proprietary Affymetrix GCOS software and the Bioconductor package affyPLM.¹⁵ If replicate arrays were available from individual samples, the data set with the best quality control assessment was selected for analysis. Preprocessing of the raw probe-level intensity data was performed using the GCRMA algorithm.¹⁶

### Proteomic Analysis

A multiplex analysis of 97 proteins (HumanMAP version 1.6 plus amphiregulin, betacellulin, EGFR, heparin-binding (HB)-EGF, epiregulin, interleukin-18, transforming growth factor (TGF)-α, and thrombospondin-1) from plasma using the Luminex xMAP® technology platform (as described in the Supplementary Methods section) was performed at Rules-Based Medicine (Austin, Texas, US). Betacellulin, EGFR and HB-EGF were only assessed in 23 samples from patients who were enrolled later in the trial.

### Statistical analysis

Response rates and progression-free survival (PFS), defined as the duration from the first infusion of cetuximab until the first radiologically confirmed disease progression under the combination of cetuximab and FOLFIRI, for patients whose tumors were wild-type or mutant with respect to *KRAS* were compared using Fisher's exact and logrank tests, respectively.

All statistical analyses of the IHC, microarray and proteomics data (see Supplementary Methods) were performed using Bioconductor software¹⁵ and SAS version 9.1. These exploratory analyses were viewed as hypothesis-generating.

### REFERENCES (relevant and/or used in this description)

1. Cunningham D, Humblet Y, Siena S, et al: Cetuximab monotherapy and cetuximab plus irinotecan in irinotecan-refractory metastatic colorectal cancer. N Engl J Med 351:337-345,2004
2. Jonker DJ, O'Callaghan CJ, Karapetis CS, et al: Cetuximab for the treatment of colorectal cancer. N Engl J Med 357:2040-2048, 2007
3. Sobrero AF, Maurel J, Fehrenbacher L, et al: EPIC: phase III trial of cetuximab plus irinotecan after fluoropyrimidine and oxaliplatin failure in patients with metastatic colorectal cancer. J Clin Oncol 26:2311-2319, 2008
4. Van Cutsem E, Kohne CH, Hitre E, et al: Cetuximab and chemotherapy as initial treatment for metastatic colorectal cancer. N Engl J Med 360:1408-1417, 2009
5. Cappuzzo F, Finocchiaro G, Rossi E, et al: EGFR FISH assay predicts for response to cetuximab in chemotherapy refractory colorectal cancer patients. Ann Oncol 19:717-723, 2008
6. Chung KY, Shia J, Kemeny NE, et al: Cetuximab Shows Activity in Colorectal Cancer Patients With Tumors That Do Not Express the Epidermal Growth Factor Receptor by Immunohistochemistry. J Clin Oncol 23:1803-1810, 2005
7. De Roock W, Piessevaux H, De Schutter J, et al: KRAS wild-type state predicts survival and is associated to early radiological response in metastatic colorectal cancer treated with cetuximab. Ann Oncol 19:508-515, 2008
8. Di Fiore F, Blanchard F, Charbonnier F, et al: Clinical relevance of KRAS mutation detection in metastatic colorectal cancer treated by cetuximab plus chemotherapy. Br J Cancer 96:1166-1169, 2007
9. Finocchiaro G, Cappuzzo F, Janne PA, et al: EGFR, HER2 and Kras as predictive factors for cetuximab sensitivity in colorectal cancer. J Clin Oncol 25, 2007:(suppl; abstr 4021)
10. Khambata-Ford S, Garrett CR, Meropol NJ, et al: Expression of epiregulin and amphiregulin and K-ras mutation status predict disease control in metastatic colorectal cancer patients treated with cetuximab. J Clin Oncol 25:3230-3237, 2007
11. Lievre A, Bachet JB, Boige V, et al: KRAS mutations as an independent prognostic factor in patients with advanced colorectal cancer treated with cetuximab. J Clin Oncol 26:374-379, 2008
12. Bokemeyer C, Bondarenko I, Makhson A, et al: Fluorouracil, leucovorin, and oxaliplatin with and without cetuximab in the first-line treatment of metastatic colorectal cancer. J Clin Oncol 27:663-671, 2009
13. Karapetis CS, Khambata-Ford S, Jonker DJ, et al: K-ras mutations and benefit from cetuximab in advanced colorectal cancer. N Engl J Med 359:1757-1765, 2008
14. Rojo F, Tabernero J, Albanell J, et al: Pharmacodynamic studies of gefitinib in tumor biopsy specimens from patients with advanced gastric carcinoma. J Clin Oncol 24:4309-4316, 2006
15. Chen CY, Shiesh SC, Wu SJ: Rapid detection of K-ras mutations in bile by peptide nucleic acid-mediated PCR clamping and melting curve analysis: comparison with restriction fragment length polymorphism analysis. Clin Chem 50:481-489, 2004
16. Gentleman RC, Carey VJ, Bates DM, et al: Bioconductor: open software development for computational biology and bioinformatics. Genome Biol 5:R80, 2004
17. Wu Z, Irizarry RA, Gentleman R, et al: A model-based background adjustment for oligonucleotide expression arrays. JASA 99:909-917, 2004
18. Di Nicolantonio F, Martini M, Molinari F, et al: Wild-type BRAF is required for response to panitumumab or cetuximab in metastatic colorectal cancer. J Clin Oncol 26:5705-5712, 2008
19. Perrone F, Lampis A, Orsenigo M, et al: PI3KCA/PTEN deregulation contributes to impaired responses to cetuximab in metastatic colorectal cancer patients. Ann Oncol 20:84-90,2009
20. Jhawer M, Goel S, Wilson AJ, et al: PIK3CA mutation/PTEN expression status predicts response of colon cancer cells to the epidermal growth factor receptor inhibitor cetuximab. Cancer Res 68:1953-1961, 2008
21. Loupakis F, Pollina L, Stasi I, et al: PTEN Expression and KRAS Mutations on Primary Tumors and Metastases in the Prediction of Benefit From Cetuximab Plus Irinotecan for Patients With Metastatic Colorectal Cancer. J Clin Oncol 27:2622-2629, 2009
22. Vallbohmer D, Zhang W, Gordon M, et al: Molecular determinants of cetuximab efficacy. J Clin Oncol 23:3536-3544, 2005
23. Vincenzi B, Santini D, Russo A, et al: Circulating VEGF reduction, response and outcome in advanced colorectal cancer patients treated with cetuximab plus irinotecan. Pharmacogenomics 8:319-327, 2007
24. Lurje G, Nagashima F, Zhang W, et al: Polymorphisms in cyclooxygenase-2 and epidermal growth factor receptor are associated with progression-free survival independent of K-ras in metastatic colorectal cancer patients treated with single-agent cetuximab. Clin Cancer Res 14:7884-7895, 2008
25. Oden-Gangloff A, Di Fiore F, Bibeau F, et al: TP53 mutations predict disease control in metastatic colorectal cancer treated with cetuximab-based chemotherapy. Br J Cancer 100:1330-1335, 2009
26. Hsu DS, Balakumaran BS, Acharya CR, et al: Pharmacogenomic strategies provide a rational approach to the treatment of cisplatin-resistant patients with advanced cancer. J Clin Oncol 25:4350-4357, 2007
27. Bonnefoi H, Potti A, Delorenzi M, et al: Validation of gene signatures that predict the response of breast cancer to neoadjuvant chemotherapy: a substudy of the EORTC 10994/BIG 00-01 clinical trial. Lancet Oncol 8:1071-1078, 2007
28. Burington B, Barlogie B, Zhan F, et al: Tumor cell gene expression changes following short-term in vivo exposure to single agent chemotherapeutics are related to survival in multiple myeloma. Clin Cancer Res 14:4821-4829, 2008
29. Kunz M: Genomic signatures for individualized treatment of malignant tumors. Curr Drug Discov Technol 5:9-14, 2008
30. de Reynies A, Boige V, Milano G, et al: KRAS mutation signature in colorectal tumors significantly overlaps with the cetuximab response signature. J Clin Oncol 26:2228-2230; author reply 2230-2221, 2008
31. Tejpar S, De Roock W, Biesmans B, et al: High amphiregulin and epiregulin expression in KRAS wild type colorectal primaries predicts response and survival benefit after treatment with cetuximab and irinotecan for metastatic disease. ASCO Gastrointestinal Cancers Symposium, January 25-27, 2008, Orlando, FL (abstr 411)
32. Waugh DJ, Wilson C: The interleukin-8 pathway in cancer. Clin Cancer Res 14:6735-6741, 2008

## Claims

1. An *in vitro* method for predicting the likelihood that a patient suffering from KRAS wild type EGFR expressing colorectal cancer (CRC), metastatic colorectal cancer (mCRC), who is a candidate for treatment with an EGFR antibody, will respond to the treatment with said anti-EGFR antibody, comprising determining the expression level of prognostic genes or gene expression products thereof in a tissue sample, obtained from said patient by subjecting a nucleic acid sample from the tumor sample from the patient to PCR or an RNA or DNA array or a comparable diagnostic tool or apparatus, wherein
(i) high expression compared to a reference value of the gene or gene product selected from the group of genes consisting of: ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2,VAV3, ZNF337, EPDR1, KCNK5, KHDRBS3, PGM2L1, STK38, and SHROOM2 indicates that the patient is likely to respond to said treatment, and
(ii) high expression compared to a reference value of the gene or the gene product selected from the group of genes consisting of: C7orf46, CAST, DCP2, DIP2B, ERAP1, INSIG2, KIF21A, KLK6, NGRN, NRIP1, PHGDH, PPP1R9A, QPCT, RABEP1, RPA1,RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, and UBE2K, indicates that the patient is likely not to respond to said treatment, wherein the reference value is defined by one or more of a specific functional or clinical property, and / or a specific expression profile and is obtained from a reference patient or patient group that does not express or express little of said gene or gene product, wherein said prognostic genes or gene expression products thereof include at least VAV3 and TGFa.

2. A method of claim 1, wherein the treatment with said anti-EGFR antibody is a first-line therapy and the genes or gene expression products selected from said group (i) are one or more of of ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, VAV3 and ZFYVE26, and from group (ii) are one or more of C7orf46, CAST, DCP2, DIP2B, ERAP1, INSIG2, KIF21A, KLK6, NGRN, NRIP1, PHGDH, PPP1R9A, QPCT, RABEP1, RPA1,RPL22L1, SKP1, SLC25A27, SLC25A46, SOCS6, TPD52, TGFA, ZDHHC2, and ZNF654.

3. A method of claim 1, wherein the treatment with said anti-EGFR antibody is a combination therapy with a chemotherapeutic agent after the patient has developed a chemo-refractory tumor, and the selected genes or gene expression products from group (i) are one or more of RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL,NOSIP, PITX2, POF1B, PPARG, PPP1R14C, PRR15, PSMG1, RAB15, RAB40B, RNF43, RPS23, SLC44A3, SOX4, THEM2,VAV3, ZNF337, EPDR1, KCNK5, KHDRBS3, PGM2L1, STK38, and SHROOM2, and from group (ii) are one or more of ASB6, ATM, BM11, CDC42EP2, EDEM3, PLLP, RALBP1, SLC4A11, TNFSF15, TPK1, C11 orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, and UBE2K.

4. A method of claim 3, wherein the clinical overall response (OR) is measured as partial response versus stable or progressive disease, and the selected genes from group (i) are one or more of VAV3, RGMB, and SPIRE2, and from group (ii) are one or more of ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFa, VAPA, and UBE2K, or the respective gene expression products of each of said groups.

5. A method of any of the claims 1 - 4, wherein the reference value is an expression threshold value of a control gene or the ratio of gene expression of selected genes from group (i) in comparison to gene expression of selected genes from group (ii) or the reference value is an expression threshold value defined by specific clinical response parameters to be determined or by specific pre-treatment or treatment conditions, wherein the clinical response parameter is progression free survival time (PFS), overall survival time (OS), partial response (PR), stable disease (SD), progressive disease (PD) or combinations thereof.

6. A method of any of the claims 1 - 5, wherein the tissue samples taken from the patient before treatment or on treatment with said anti-EGFR antibody are used.

7. A method of claim 6, wherein the expression levels of the genes or gene expression products obtained on treatment are compared with the values obtained before starting treatment of said patient.

8. An *in vitro* method for predicting the likelihood that a patient suffering from KRAS wild type EGFR expressing colorectal cancer (CRC), metastatic colorectal cancer (mCRC), will respond therapeutically to the treatment with an anti-EGFR antibody, the method comprises:
(a) measuring by diagnostic means and / or diagnostic apparatus in a biopsy tissue sample from tumor tissue or plasma of said patient the expression level of biomarkers selected from the group (i) consisting of ADAMDEC1, BSDC1, C1 orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1 B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2,VAV3, ZNF337, EPDR1, KCNK5, KHDRBS3, PGM2L1, STK38, SHROOM2,
and / or from group (ii) consisting of C7orf46, CAST, DCP2, DIP2B, ERAP1, INSIG2, KIF21A, KLK6, NGRN, NRIP1, PHGDH, PPP1R9A, QPCT, RABEP1, RPA1,RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11 orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, and UBE2K,
(b) exposing ex-vivo a tissue sample from tumor or plasma of said patient to said anti-EGFR antibody, (c) measuring again in said exposed tissue sample of step (b) the expression level of one or more biomarkers specified in step (a), and (d) calculating the differences in expression levels measured in steps(b) and (c), wherein an increase in the expression level of the biomarkers of group (i) obtained in step (c) compared to step (a) indicates an increased likelihood that said patient responds therapeutically to the treatment with said anti-EGFR antibody, and wherein an increase in the expression level of the biomarkers of group (ii) obtained in step (c) compared to step (a) indicates a decreased likelihood that said patient responds therapeutically to the treatment with said anti-EGFR antibody, wherein said biomarkers include at least VAV3 and TGFa.

9. A method of any of the claims 1 -8, wherein additionally the expression levels of the biomarkers AREG or EREG are determined

10. The method of claim 9, wherein the expression levels of the following biomarkers are determined:
(i) VAV3, TGFA, AREG and optionally EGF, or
(ii) VAV3, TGFA, EREG and optionally EGF.

11. A method of any of the claims 1 - 10, wherein the patient suffering from a KRAS wild type EGFR expressing tumor additionally has a EGFR mutation in tumor tissue.

12. A method of claim 11, wherein the EGFR mutation is a R521K polymorphism

13. A method of any of the claims 1-12, wherein the anti-EGFR antibody is c225 (cetuximab).

14. Use of the genetic biomarkers comprising the biomarkers selected from the group consisting of ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2,VAV3, ZNF337, EPDR1, KCNK5, KHDRBS3, PGM2L1, STK38, SHROOM2,
C7orf46, CAST, DCP2, DIP2B, ERAP1, INSIG2, KIF21A, KLK6, NGRN, NRIP1, PHGDH, PPP1R9A, QPCT, RABEP1, RPA1,RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11 orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, and UBE2K, optionally in combination with AREG and /or EREG for predicting the pharmaceutical efficacy and / or clinical response of a patient suffering from KRAS wild type EGFR expressing colorectal cancer (CRC), metastatic colorectal cancer (mCRC) to an anti-EGFR antibody intended to be used for treatment, wherein said prediction results from calculating the differences in expression levels towards a threshold value to be determined from the underlying clinical determination parameters, wherein said genetic biomarkers include at least VAV3 and TGFa.

15. Use of claim 14, wherein said protein expression product is determined from a body fluid of the patient including plasma.

16. Use of any of the claims 14 or 15, wherein the intended underlying treatment is a first-line treatment.

17. Use of any of the claims 14 - 16, wherein the intended underlying treatment is a combination treatment of said anti-EGFR antibody with a chemotherapeutic agent, and said patient has developed chemo-refractory cancer

18. Use according to any of the claims 14-17, wherein the anti-EGFR antibody is c225 (cetuximab).

## Patentansprüche

1. *In vitro*-Verfahren zur Vorhersage der Wahrscheinlichkeit, dass ein Patient, der an KRAS-Wildtyp-EGFR-exprimierendem Kolorektalkrebs (CRC), metastatischem Kolorektalkrebs (mCRC), leidet, der ein Kandidat zur Behandlung mit einem EGFR-Antikörper ist, auf die Behandlung mit dem anti-EGFR-Antikörper anspricht, umfassend das Bestimmen des Expressionsniveaus prognostischer Gene oder Genexpressionsprodukte davon in einer Gewebeprobe, die aus dem Patient erhalten wurde, indem eine Nukleinsäureprobe aus der Tumorprobe des Patienten einer PCR oder einem RNA- oder DNA-Array oder einem vergleichbaren Diagnosewerkzeug oder -Gerät unterworfen wird, wobei
(i) eine hohe Expression im Vergleich zu einem Referenzwert des Gens oder Genprodukts, ausgewählt aus der Gruppe von Genen bestehend aus: ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38 und SHROOM2, anzeigt, dass der Patient wahrscheinlich auf die Behandlung anspricht, und
(ii) eine hohe Expression im Vergleich zu einem Referenzwert des Gens oder Genprodukts, ausgewählt aus der Gruppe von Genen bestehend aus: C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA und UBE2K, anzeigt, dass der Patient wahrscheinlich nicht auf die Behandlung anspricht, wobei der Referenzwert durch eine oder mehrere einer spezifischen funktionellen oder klinischen Eigenschaft und/ oder eines spezifischen Expressionsprofils definiert ist und aus einem Referenzpatient oder einer Patientengruppe erhalten wird, der/die das Gen oder Genprodukt nicht exprimiert oder geringfügig exprimiert, wobei die prognostischen Gene oder Genexpressionsprodukte davon zumindest VAV3 und TGFa umfassen.

2. Verfahren nach Anspruch 1, wobei die Behandlung mit dem anti-EGFR-Antikörper eine Erstlinientherapie ist, und die aus der Gruppe (i) ausgewählten Gene oder Genexpressionsprodukte eine oder mehrere von ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1 B, URM1, VAV3 und ZFYVE26 sind, und aus der Gruppe (ii) eine oder mehrere von C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, TGFA, ZDHHC2 und ZNF654 sind.

3. Verfahren nach Anspruch 1, wobei die Behandlung mit dem anti-EGFR-Antikörper eine Kombinationstherapie mit einem Chemotherapeutikum ist, nachdem der Patient einen chemorefraktären Tumor entwickelt hat, und die ausgewählten Gene oder Genexpressionsprodukte aus Gruppe (i) eine oder mehrere von RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38 und SHROOM2 sind, und aus Gruppe (ii) eine oder mehrere von ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA und UBE2K sind.

4. Verfahren nach Anspruch 3, wobei die klinische Gesamtreaktion (OR) als Teilantwort gegenüber einer stabilen oder progressiven Krankheit gemessen wird, und die ausgewählten Gene aus der Gruppe (i) ein oder mehrere von VAV3, RGMB und SPIRE2 sind, und aus Gruppe (ii) ein oder mehrere von ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFa, VAPA und UBE2K sind, oder die entsprechenden Genexpressionsprodukte von jeder der Gruppen.

5. Verfahren nach einem der Ansprüche 1 bis 4, wobei der Referenzwert ein Expressionsschwellenwert eines Kontrollgens oder das Verhältnis der Genexpression ausgewählter Gene aus Gruppe (i) im Vergleich zur Genexpression ausgewählter Gene aus Gruppe (ii) ist oder wobei der Referenzwert ein Expressionsschwellenwert ist, der durch zu bestimmende spezifische klinische Reaktionsparameter oder durch spezifische Vorbehandlungs- oder Behandlungsbedingungen definiert wird, wobei es sich bei dem klinischen Reaktionsparameter um progressionsfreie Überlebenszeit (PFS), Gesamtüberlebenszeit (OS), Teilantwort (PR), stabile Krankheit (SD), progressive Krankheit (PD) oder Kombinationen davon handelt.

6. Verfahren nach einem der Ansprüche 1 bis 5, wobei die Gewebeproben, die aus dem Patienten vor der Behandlung oder bei der Behandlung mit dem anti-EGFR-Antikörper entnommen wurden, verwendet werden.

7. Verfahren nach Anspruch 6, wobei die Expressionsniveaus der Gene oder Genexpressionsprodukte, die bei Behandlung erhalten wurden, mit den Werten verglichen werden, die vor dem Beginn der Behandlung des Patienten erhalten wurden.

8. *In vitro*-Verfahren zur Vorhersage der Wahrscheinlichkeit, dass ein Patient, der an KRAS-Wildtyp-EGFR-exprimierendem Kolorektalkrebs (CRC), metastatischem Kolorektalkrebs (mCRC) leidet, therapeutisch auf die Behandlung mit einem anti-EGFR-Antikörper anspricht, wobei das Verfahren umfasst:
(a) Mittels Diagnosemittel und/oder Diagnosegerät in einer Biopsie-Gewebeprobe aus Tumorgewebe oder Plasma des Patienten erfolgendes Messen des Expressionsniveaus von Biomarkern, ausgewählt aus der Gruppe (i), bestehend aus ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1 B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, SHROOM2,
und/oder aus der Gruppe (ii), bestehend aus C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA und UBE2K,
(b) Ex-vivo-Aussetzen einer Gewebeprobe aus Tumor oder Plasma des Patienten gegenüber dem anti-EGFR-Antikörper, (c) erneutes Messen in der ausgesetzten Gewebeprobe von Schritt (b) des Expressionsniveaus von einem oder mehreren der in Schritt (a) angegebenen Biomarkern und (d) Berechnen der Unterschiede der in den Schritten (b) und (c) gemessenen Expressionsniveaus, wobei ein Anstieg des in Schritt (c) im Vergleich zu Schritt (a) erhaltenen Expressionsniveaus der Biomarker der Gruppe (i) eine erhöhte Wahrscheinlichkeit anzeigt, dass der Patient therapeutisch auf die Behandlung mit dem anti-EGFR-Antikörper anspricht, und wobei ein Anstieg des in Schritt (c) im Vergleich zu Schritt (a) erhaltenen Expressionsniveaus der Biomarker der Gruppe (ii) eine verringerte Wahrscheinlichkeit anzeigt, dass der Patient therapeutisch auf die Behandlung mit dem anti-EGFR-Antikörper anspricht, wobei die Biomarker zumindest VAV3 und TGFa umfassen.

9. Verfahren nach einem der Ansprüche 1 bis 8, wobei zusätzlich die Expressionsniveaus der Biomarker AREG oder EREG bestimmt werden.

10. Verfahren nach Anspruch 9, wobei die Expressionsniveaus der folgenden Biomarker bestimmt werden:
(i) VAV3, TGFA, AREG und gegebenenfalls EGF, oder
(ii) VAV3, TGFA, EREG und gegebenenfalls EGF.

11. Verfahren nach einem der Ansprüche 1 bis 10, wobei der Patient der an einem KRAS-Wildtyp-EGFR-exprimierenden Tumor leidet, zusätzlich eine EGFR-Mutation im Tumorgewebe aufweist.

12. Verfahren nach Anspruch 11, wobei die EGFR-Mutation ein R521K-Polymorphismus ist.

13. Verfahren nach einem der Ansprüche 1 bis 12, wobei der anti-EGFR-Antikörper c225 (Cetuximab) ist.

14. Verwendung der genetischen Biomarker, umfassend die Biomarker ausgewählt aus der Gruppe bestehend aus ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, SHROOM2,
C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA und UBE2K, gegebenenfalls in Kombination mit AREG und/oder EREG zur Vorhersage der pharmazeutischen Wirksamkeit und/oder der klinischen Reaktion eines Patienten, der an KRAS-Wildtyp-EGFR-exprimierendem Kolorektalkrebs (CRC), metastatischem Kolorektalkrebs (mCRC) leidet, gegenüber einem anti-EGFR-Antikörper, der zur Behandlung verwendet werden soll, wobei die Vorhersage aus der Berechnung der Unterschiede der Expressionsniveaus zu einem Schwellenwert herrührt, der aus den zugrundeliegenden Bestimmungsparametern bestimmt werden soll, wobei die genetischen Biomarker zumindest VAV3 und TGFa umfassen.

15. Verwendung nach Anspruch 14, wobei das Proteinexpressionsprodukt aus einer Körperflüssigkeit des Patienten einschließlich Plasma bestimmt wird.

16. Verwendung nach einem der Ansprüche 14 oder 15, wobei die vorgesehene zugrundeliegende Behandlung eine Erstlinienbehandlung ist.

17. Verwendung nach einem der Ansprüche 14 bis 16, wobei die vorgesehene zugrundeliegende Behandlung eine Kombinationsbehandlung des anti-EGFR-Antikörpers mit einem Chemotherapeutikum ist, und der Patient chemorefraktären Krebs entwickelt hat.

18. Verwendung nach einem der Ansprüche 14 bis 17, wobei der anti-EGFR-Antikörper c225 (Cetuximab) ist.

## Revendications

1. Méthode *in vitro* de prédiction de la probabilité qu'un patient souffrant d'un cancer colorectal (CRC) avec KRAS de type sauvage exprimant l'EGFR, d'un cancer colorectal métastatique (mCRC), qui est un candidat pour un traitement par un anticorps anti-EGFR, répondra au traitement par ledit anticorps anti-EGFR, comprenant la détermination du taux d'expression de gènes de pronostic ou de produits d'expression génique de ceux-ci dans un échantillon de tissu, obtenu à partir dudit patient en soumettant un échantillon d'acide nucléique issu de l'échantillon de tumeur du patient à une PCR ou une puce à ARN ou ADN ou un outil ou appareil comparable de diagnostic, dans laquelle
(i) une expression élevée par rapport à une valeur de référence du gène ou du produit de gène choisi dans le groupe de gènes constitué par : ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, et SHROOM2 indique que le patient répondra probablement audit traitement, et
(ii) une expression élevée par rapport à une valeur de référence du gène ou du produit de gène choisi dans le groupe de gènes constitué par : C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11 orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIMB, TSC22D2, TGFA, VAPA, et UBE2K, indique que le patient ne répondra probablement pas audit traitement, où la valeur de référence est définie par un ou plusieurs parmi une propriété fonctionnelle ou clinique spécifique, et/ou un profil d'expression spécifique et est obtenue à partir d'un patient ou groupe de patients de référence qui n'exprime pas ou exprime peu ledit gène ou produit de gène, où lesdits gènes de pronostic ou produits d'expression génique de ceux-ci comportent au moins VAV3 et TGFa.

2. Méthode selon la revendication 1, dans laquelle le traitement par ledit anticorps anti-EGFR est une thérapie de première intention et les gènes ou produits d'expression génique choisis parmi ledit groupe (i) sont un ou plusieurs parmi ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, VAV3 et ZFYVE26, et parmi le groupe (ii) sont un ou plusieurs parmi C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, TGFA, ZDHHC2, et ZNF654.

3. Méthode selon la revendication 1, dans laquelle le traitement par ledit anticorps anti-EGFR est une thérapie associative avec un agent chimiothérapeutique après que le patient a développé une tumeur chimio-réfractaire, et les gènes ou produits d'expression génique sélectionnés issus du groupe (i) sont un ou plusieurs parmi RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, et SHROOM2, et issus du groupe (ii) sont un ou plusieurs parmi ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, et UBE2K.

4. Méthode selon la revendication 3, dans laquelle la réponse globale clinique (OR) est mesurée comme réponse partielle par rapport à une maladie stable ou progressive, et les gènes sélectionnés issus du groupe (i) sont un ou plusieurs parmi VAV3, RGMB, et SPIRE2, et issus du groupe (ii) sont un ou plusieurs parmi ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFa, VAPA, et UBE2K, ou les produits d'expression génique respectifs de chacun parmi lesdits groupes.

5. Méthode selon l'une quelconque des revendications 1-4, dans laquelle la valeur de référence est une valeur de seuil d'expression d'un gène témoin ou le rapport d'expression génique de gènes sélectionnés issus du groupe (i) par rapport à l'expression génique de gènes sélectionnés issus du groupe (ii) ou la valeur de référence est une valeur de seuil d'expression définie par des paramètres de réponse clinique spécifiques devant être déterminés ou par des conditions spécifiques de prétraitement ou de traitement, où le paramètre de réponse clinique est la durée de survie sans progression (PFS), la durée de survie globale (OS), la réponse partielle (PR), la maladie stable (SD), la maladie progressive (PD) ou des combinaisons de ceux-ci.

6. Méthode selon l'une quelconque des revendications 1-5, dans laquelle les échantillons de tissu prélevés chez le patient avant le traitement ou lors du traitement par ledit anticorps anti-EGFR, sont utilisés.

7. Méthode selon la revendication 6, dans laquelle les taux d'expressions des gènes ou produits d'expression génique obtenus lors du traitement sont comparés aux valeurs obtenues avant de commencer le traitement dudit patient.

8. Méthode *in vitro* de prédiction de la probabilité qu'un patient souffrant d'un cancer colorectal (CRC) avec KRAS de type sauvage exprimant l'EGFR, d'un cancer colorectal métastatique (mCRC), répondra thérapeutiquement au traitement par un anticorps anti-EGFR, la méthode comprenant :
(a) la mesure par un moyen de diagnostic et/ou un appareil de diagnostic dans un échantillon de tissu de biopsie issu d'un tissu tumoral ou de plasma dudit patient, du taux d'expression de biomarqueurs choisi dans le groupe (i) constitué par ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, SHROOM2,
et/ou dans le groupe (ii) constitué par C7orf46, CAST, DCP2, DIP2B, ERAP1, INSIG2, KIF21A, KLK6, NGRN, NRIP1, PHGDH, PPP1R9A, QPCT, RABEP1, RPA1, RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, et UBE2K,
(b) l'exposition *ex vivo* d'un échantillon de tissu issu d'une tumeur ou de plasma dudit patient audit anticorps anti-EGFR,
(c) la mesure de nouveau dans ledit échantillon de tissu exposé de l'étape (b) du taux d'expression d'un ou plusieurs biomarqueurs spécifiés dans l'étape (a), et
(d) le calcul des différences de taux d'expression mesurés dans les étapes (b) et (c), où une augmentation du taux d'expression des biomarqueurs issus du groupe (i) obtenu dans l'étape (c) par rapport à l'étape (a) indique une probabilité accrue que ledit patient réponde thérapeutiquement au traitement par ledit anticorps anti-EGFR, et où une augmentation du taux d'expression des biomarqueurs issus du groupe (ii) obtenu dans l'étape (c) par rapport à l'étape (a) indique une probabilité réduite que ledit patient réponde thérapeutiquement au traitement par ledit anticorps anti-EGFR, où lesdits biomarqueurs comportent au moins VAV3 et TGFa.

9. Méthode selon l'une quelconque des revendications 1-8, dans laquelle, en outre, les taux d'expression des biomarqueurs AREG ou EREG sont déterminés

10. Méthode selon la revendication 9, dans laquelle les taux d'expression des biomarqueurs suivants sont déterminés :
(i) VAV3, TGFA, AREG et éventuellement EGF, ou
(ii) VAV3, TGFA, EREG et éventuellement EGF.

11. Méthode selon l'une quelconque des revendications 1-10, dans laquelle le patient souffrant d'une tumeur avec KRAS de type sauvage exprimant l'EGFR possède en outre une mutation d'EGFR dans le tissu tumoral.

12. Méthode selon la revendication 11, dans laquelle la mutation d'EGFR est un polymorphisme R521K.

13. Méthode selon l'une quelconque des revendications 1-12, dans laquelle l'anticorps anti-EGFR est c225 (cetuximab).

14. Utilisation des biomarqueurs génétiques comprenant les biomarqueurs choisis dans le groupe constitué par ADAMDEC1, BSDC1, C1orf144, CAPZB, CDC42, DHCR7, DNAJC8, ECSIT, EXOSC10, FADS1, GBA, GLT25D1, GOSR2, IMPDH1, KLHL21, KPNA6, KPNB1, LSM12, MAN1B1, MIDN, PPAN, SH3BP2, SQLE, SSH3, TNFRSF1B, URM1, ZFYVE26, RGMB, SPIRE2, ABCC5, ACSL5, AOAH, AXIN2, CD24, CEACAM5, CEACAM6, ETS2, FMNL2, GPSM2, HDAC2, JUN, ME3, MED17, MYB, MYC, NEBL, NOSIP, PITX2, POF1B, PPARG, PPP1R14C, PRR15, PSMG1, RAB15, RAB40B, RNF43, RPS23, SLC44A3, SOX4, THEM2, VAV3, ZNF337, EPDR1, KCNK5, KHDRBS3, PGM2L1, STK38, SHROOM2,
C7orf46, CAST, DCP2, DIP2B, ERAP1, INSIG2, KIF21A, KLK6, NGRN, NRIP1, PHGDH, PPP1 R9A, QPCT, RABEP1, RPA1, RPL22L1, SKP1, SLC25A27, SLC25A46, SOCS6, TPD52, ZDHHC2, ZNF654, ASB6, ATM, BMI1, CDC42EP2, EDEM3, PLLP, RALBP1, SLC4A11, TNFSF15, TPK1, C11orf9, C1QC, CABLES1, CDK6, EHBP1, EXOC6, EXT1, FLRT3, GCNT2, MTHFS, PIK3AP1, ST3GAL1, TK2, ZDHHC14, ARFGAP3, AXUD1, CAPZB, CHSY1, DNAJB9, GOLT1B, HSPA5, LEPROTL1, LIMS1, MAPK6, MYO6, PROSC, RAB8B, RAP2B, RWDD2B, SERTAD2, SOCS5, TERF2IP, TIAL1, TIPARP, TRIM8, TSC22D2, TGFA, VAPA, et UBE2K, éventuellement en combinaison avec AREG et/ou EREG, pour la prédiction de l'efficacité pharmaceutique et/ou de la réponse clinique d'un patient souffrant d'un cancer colorectal (CRC) avec KRAS de type sauvage exprimant l'EGFR, d'un cancer colorectal métastasique (mCRC) vis-à-vis d'un anticorps anti-EGFR prévu pour être utilisé comme traitement, où ladite prédiction résulte du calcul des différences de taux d'expression vis-à-vis d'une valeur seuil à déterminer à partir des paramètres de détermination clinique sous-jacents, où lesdits biomarqueurs génétiques comportent au moins VAV3 et TGFa.

15. Utilisation selon la revendication 14, dans laquelle ledit produit d'expression de protéine est déterminé à partir d'un fluide corporel du patient, y compris le plasma.

16. Utilisation selon l'une quelconque des revendications 14 ou 15, dans laquelle le traitement sous-jacent prévu est un traitement de première intention.

17. Utilisation selon l'une quelconque des revendications 14-16, dans laquelle le traitement sous-jacent prévu est un traitement associatif dudit anticorps anti-EGFR avec un agent chimiothérapeutique, et ledit patient a développé un cancer chimio-réfractaire.

18. Utilisation selon l'une quelconque des revendications 14-17, dans laquelle l'anticorps anti-EGFR est c225 (cetuximab).
